# EUROPEAN PATENT APPLICATION

(11) **EP 4 667 511 A2**
(43) Date of publication of application: **24.12.2025**
(21) Application number: 25214103.1
(22) Date of filing: 01.04.2022
(51) Int. Cl.: C08G 83/00

(54) **DENDRITIC ARCHITECTURES AS NONVIRAL VECTORS IN GENE DELIVERY**

(30) Priority: 02.04.2021 US 202163170119 P
(62) Divisional of application: 22782290.5
(71) Applicant: Tiba Biotech LLC, Cambridge, Massachusetts 02142 (US)
(72) Inventor: TALUKDER, Poulami, Woburn, 01801 (US); RUPING, Karl, Cambridge, 02142 (US)
(74) Representative: Murgitroyd & Company

(57) **Abstract**

Disclosed herein are novel dendritic structures that can be used in combination with other lipid components to form nanoparticles with nucleic acids, to facilitate the intracellular delivery of nucleic acids both *in vitro* and *in vivo.* Nanoparticle compositions comprising the compounds and methods for their use for treating or preventing diseases or conditions are also provided.

## Description

### CROSS-REFERENCE TO RELATED APPLICATIONS

This application claims the benefit of U.S. provisional patent application No. 63/170,119, which was filed April 2, 2021, was titled "DENDRITIC ARCHITECTURES FOR NUCLEIC ACID DELIVERY," and is incorporated herein by reference as if fully set forth.

### FIELD

The disclosure relates to dendritic structures as nonviral vectors or carriers for efficient delivery of nucleic acids to a subject for treating or preventing diseases and/or disorders, and nanoparticle compositions comprising the carriers and nucleic acids. The disclosure also relates to methods of formulating the nanoparticle compositions and methods of treating diseases and/or disorders in the subjects with such nanoparticle compositions.

### BACKGROUND

Nucleic acid vaccines and therapeutics have emerged as a promising approach to prevent and treat several diseases or conditions. Certain nucleic acids are stable for only a limited duration in cells or plasma, and their highly charged nature make the delivery even more complicated across cell membranes. Compositions that can stabilize and deliver such agents into cells are therefore of particular interest. (Mendes et al., 2017, Molecules 22(9), 1401; Jones et al., 2013, Mol. Pharmaceutics 10, 4082-4098; and Nishikawa and Huang, 2001 Hum. Gene Ther. 12, 861-870). Nucleic acid delivery vectors ideally should overcome different extra- and intracellular barriers such as nucleic acid degradation by endonucleases, cellular internalization, endosomal escape, payload release from the vector and access to the desired target, all of with minimal toxicity. (Jones et al., 2013, Mol. Pharmaceutics 10, 4082-4098; Nishikawa and Huang, 2001 Hum. Gene Ther. 12, 861-870; Gomes et al., 2014 MRS Bull. 39, 60-70; and Dufes et al., 2005, Adv. Drug Delivery Rev. 57, 2177-2202).

Although Lipid nanoparticle (LNP) systems represent the most advanced delivery systems for genetic drugs because of their high nucleic acid encapsulation efficiency and potent transfection etc., mild to moderate local and systemic adverse events have been observed (Mol Ther Nucleic Acids. 2019 Apr 15; 15: 1-11; Ther Deliv. 2016;7(5):319-334. doi:10.4155/tde-2016-0006).

Polyester dendrons called have been reported, which have building blocks known to be biocompatible or degradable to natural metabolites *in vivo* (Carnahan and Grinstaff, 2001, J. Am. Chem. Soc. 123, 2905; Carnahan and Grinstaff, 2001, Macromolecules 34, 7648; and Carnahan and Grinstaff, 2006, Macromolecules 39, 609).

### SUMMARY

In an aspect, the invention relates to a nucleic acid carrier comprising a dendron or dendrimer of Formula Ia, Ib, Ic, Id, Ie or If: 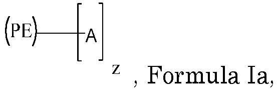 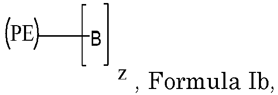 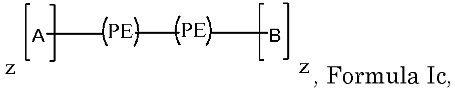 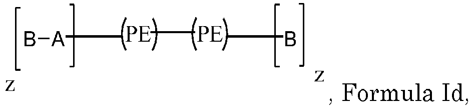 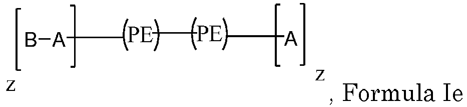 or 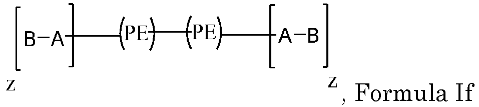 wherein PE is a polyester (PE) dendron which includes a core and a plurality of monomeric polyester units that form one or more generations, A is an amine, B is a hydrophobic unit and z is the number of surface groups.

In an aspect, the invention relates to a nanoparticle composition comprising any one of the nucleic acid carriers disclosed herein, a therapeutic or immunogenic nucleic acid agent enclosed therein, and a conjugated lipid. In an aspect, the invention relates to a nanoparticle composition comprising any one of the nucleic acid carriers disclosed herein, a therapeutic or immunogenic nucleic acid agent enclosed therein, one conjugated lipid (e.g., PEG-Lipid) disclosed herein, and a mixture of a phospholipid and cholesterol or a derivative thereof to improve with intracellular delivery as well as nanoparticle stability *in vivo*.

In an aspect, the invention relates to a method for treating or preventing a disease or condition in a subject. The method involves providing any one of the nanoparticle compositions disclosed herein and administering a therapeutically effective amount of these nanoparticle compositions to a subject.

### BRIEF DESCRIPTION OF THE DRAWINGS

The following detailed description of preferred embodiments of the present invention will be better understood when read in conjunction with the appended drawings. For the purpose of illustrating the invention, particular embodiments are shown in the drawings. It is understood, however, that the invention is not limited to the precise arrangements and instrumentalities shown. In the drawings:
FIG. 1 illustrates Dynamic Light Scattering (DLS) analysis showing size distribution profiles of the nanoparticle composition (PE-G2-Hexadecyl-propylpyrrolidine as nucleic acid carrier, formulated at pH 5.0)
FIG. 2 illustrates Dynamic Light Scattering (DLS) analysis showing size distribution profiles of the nanoparticle composition (PE-G2-Hexadecyl-propylpyrrolidine as nucleic acid carrier, formulated at pH 6.0)
FIG. 3 illustrates a photograph of the agarose gel showing the binding of the PE-G2-Hexadecyl-propylpyrrolidine with RNA. The gels were stained with ethidium bromide (EB) and gel images were taken on a Syngene G Box imaging system (Syngene, USA).
FIG. 4 illustrates *in* vivo secreted alkaline phosphatase (SEAP) expression for the SEAP Replicon RNA formulated into PE-G2-Hexadecyl-propylpyrrolidine nanoparticles and collected from mice 1 day, 4 days and 6 days after administration.
FIG. 5 illustrates Dynamic Light Scattering (DLS) analysis showing size distribution profiles of the nanoparticle composition (PE-G2-A1-Ricinoleic.PE-G2-Linoleic as nucleic acid carrier, formulated at pH 5.0)
FIG. 6 illustrates a photograph of the agarose gel showing the binding of the PE-G2-A1-Ricinoleic.PE-G2-Linoleic with RNA. The gels were stained with ethidium bromide (EB) and gel images were taken on a Syngene G Box imaging system (Syngene, USA).
FIG. 7 shows *in vitro* SEAP expression via quantiblue assay for nanoparticles containing SEAP Replicon RNA and PE-G2-A1-Ricinoleic.PE-G2-Linoleic.
FIGS. 8A and 8B illustrate Dynamic Light Scattering (DLS) analysis showing size distribution profiles of the nanoparticle composition containing PE-G1-A1-Ricinoleic. PE-G1-A1-Ricinoleic or PE-G2-A1-Ricinoleic. PE-G1-A1-Ricinoleic as nucleic acid carrier and SEAP Replicon RNA.
FIG. 9A shows *in vitro* SEAP expression via quantiblue assay for nanoparticles containing SEAP Replicon RNA and PE-G1-A1-Ricinoleic. PEG1-A1-Ricinoleic or PE-G2-A1-Ricinoleic. PE-G1-A1-Ricinoleic.
FIG. 9B illustrates the *in* vivo SEAP expression for nanoparticles containing SEAP Replicon RNA and PE-G1-A1-Ricinoleic. PE-G1-A1-Ricinoleic or PE-G2-A1-Ricinoleic. PE-G1-A1-Ricinoleic and collected from mice 3 days after administration.
FIG. 10 illustrates endpoint dilution titers for mouse serum IgG specific to SARS-CoV-2 Spike protein after vaccination with SARS-CoV-2 Spike replicon RNA formulated with PE-G1-A1-Ricinoleic. PE-G1-A1-Ricinoleic or PE-G2-A1-Ricinoleic. PE-G1-A1-Ricinoleic delivery materials.

### DETAILED DESCRIPTION

Certain terminology is used in the following description for convenience only and is not limiting.

The term "substitute" refers to the ability to change one functional group, or a moiety included therein, for another functional group on a molecule provided that the valency of all atoms is maintained on the parent structure. The substituted group is interchangeably referred herein as "substitution" or "substituent." When more than one position in any given structure is substituted with more than one substituent selected from a specified group, the substituent may be either the same or different at every position.

The term "Amines" are formally derivatives of ammonia, wherein one or more hydrogen atoms have been replaced by a substituent such as an alkyl group.

The term "surface groups" as used herein, means terminal groups on the surface of dendritic molecules. The surface of the nucleic acid carriers is modified with hydrophobic tails (described as component "B" herein) or amines (described as component "A" herein) in order to assist self-assembly properties and condense nucleic acids.

The term "alkyl as used herein, means a straight or branched chain hydrocarbon containing from 1 to 28 carbon atoms. Alkyl chain length may be useful for controlling hydrophobicity and self-assembly property of nucleic acid carrier. Representative examples of alkyl include, but are not limited to, methyl, ethyl, n-propyl. iso-propyl. n-butyl, sec-butyl, iso-butyl, tert-butyl, n-pentyl, isopentyl, neopentyl, n-hexyl, 3-methylhexyl, 2,2-dimethyl pentyl, 2,3-dimethylpentyl, n-heptyl, n-octyl, n-nonyl, and n-decyl.

The words "a" and "one," as used in the claims and in the corresponding portions of the specification, are defined as including one or more of the referenced item unless specifically stated otherwise. This terminology includes the words above specifically mentioned, derivatives thereof, and words of similar import. The phrase "at least one" followed by a list of two or more items, such as "A, B, or C," means any individual one of A, B or C as well as any combination thereof.

Non-viral vectors have the potential to address many limitations associated with viral vectors, including carcinogenesis, immunogenicity, and broad tropism. A "nanoparticle composition" refers to a composition that includes a nucleic acid carrier and a nucleic acid payload. The nucleic acid payload is "carried" by the nucleic acid carrier, and may be enclosed by the nucleic acid carrier.

An embodiment comprises a polyester dendron (a non-limiting example of which is a 2,2-bismethylolpropionic acid (bis-MPA) monomer based dendron) as a building block as modified with amines and hydrophobic tails so to condense nucleic acid and to self-assemble into a nanoparticle composition that protects the nucleic acid from degradation. In an embodiment, the dendron may transport the nucleic acid to a cell.

An embodiment comprises a nucleic acid carrier having the formula Ia, Ib, Ic, Id, Ie or If: 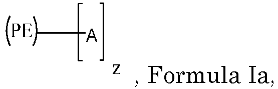 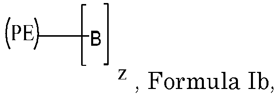 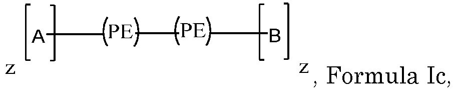 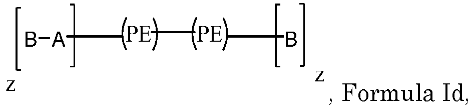 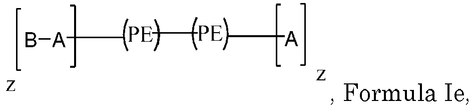 or 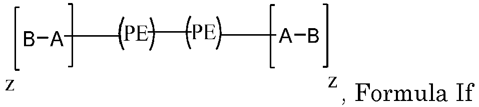 wherein PE is a polyester dendron, which comprises a core and monomeric polyester units layered around the core to form a tree-like structure, where each layer is called a generation (G), A is an amine, and B is a hydrophobic unit, and z is the number of surface groups.

PE may have Formula II:

[(core)-Gn-o] , Formula II,

G is a layer or generation of dendron; n is a generation number, whose values range from 1 to 10; and the monomeric polyester unit may be but is not limited to 2,2-bis(hydroxymethyl) propionic acid or 2,2-Bis(hydroxymethyl)butyric acid. Z has Formula III:

z = bⁿ, Formula III,

wherein b is branch point multiplicity, or number of branches at each branching point, and n is a generation number.

In the nucleic acid carrier that comprises 2,2-bis(hydroxymethyl) propionic acid or 2,2-Bis(hydroxymethyl)butyric acid as the monomeric polyester unit, branch point multiplicity or number of branches at each branching point, b, is 2.

The structure of the core may influence amine and/or charge density, diameter, and flexibility of the resultant nucleic acid carrier. These attributes may influence the physicochemical properties of a nucleic acid carrier, its interaction with nucleic acid, and gene transfer activity.

In an embodiment, the core may be selected from the following scaffolds: 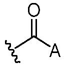 or 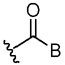 wherein A is an amine and B is a hydrophobic unit.

The amine A is a moiety that imparts proton-accepting functionality to the nucleic acid carrier molecule by containing one or more nitrogen atoms with lone pairs. The amine is thus able to accept a free proton (H+) under acidic conditions. In preferred embodiments the nitrogen atom(s) are present in the form of secondary or tertiary amines. The amine may be selected from the following moieties: 1-(2-Aminoethyl)piperazine, 1-Piperidineethanamine, 1-Dimethylamino-2-propylamine, 1-(3-Aminopropyl)pyrrolidine, 1-(2-Aminoethyl)pyrrolidine, 1-(2-Aminoethyl)piperidine, 2-(1-Piperazinyl)ethylamine, 2-(4-Methyl-piperazin-1-yl)-ethylamine, 3-(Dimethylamino)-1-propylamine, 3-(Diethylamino)propylamine, (4-Aminobutyl)dimethylamine, 4-(1-Pyrrolidinyl)-1-butylamine, 4-Morpholineethanamine, 4-Morpholinepropanamine, 4-Methyl-1-piperazineethanamine, 4-(Diethylamino)butylamine, N,N-Dimethylethylenediamine N,N-Dimethyldipropylenetriamine, N,N'-Dimethyl-N, N'-bis(3-methylaminopropyl)trimethylenediamine, N1-(2-aminoethyl)-N1-methylpropane-1,3-diamine, N1-(3-aminopropyl)-N1-methylbutane-1,4-diamine, N1-(4-aminobutyl)-N1-methylbutane-1,4-diamine, N1-(3-aminopropyl)propane-1,3-diamine, N1-(3-aminopropyl)-N1-methylpropane-1,3-diamine, 3-((3-aminopropyl)(methyl)amino)propan-1-ol, 1-Dimethylamino-2-propanol, 1-[Bis[3-(dimethylamino)propyl]amino]-2-propanol, 2-Dimethylaminoethanol, 2-(Diethylamino)ethanol, 2-[2-(Dimethylamino)ethoxy]ethanol, 2-(2-Aminoethoxy)ethanol, 2-{[2-(Dimethylamino)ethyl]methylamino}ethanol, 3-Dimethylamino-1-propanol, 3-Diethylamino-1-propanol, 4-(Dimethylamino)-1-butanol, 4-Diethylamino-2-butyn-1-ol, N-Methyldiethanolamine, 4-(diethylamino)butan-1-ol, 3-(azetidin-1-yl)propan-1-ol, 3-(pyrrolidin-1-yl)propan-1-ol, 3-(piperidin-1-yl)propan-1-ol, 4-(azetidin-1-yl)butan-1-ol, 4-(pyrrolidin-1-yl)butan-1-ol, 4-(piperidin-1-yl)butan-1-ol, 3-morpholinopropan-1-ol, 4-morpholinobutan-1-ol, 3-(4-methylpiperazin-1-yl)propan-1-ol, 4-(4-methylpiperazin-1-yl)butan-1-ol as follows.

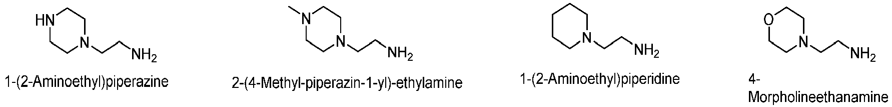

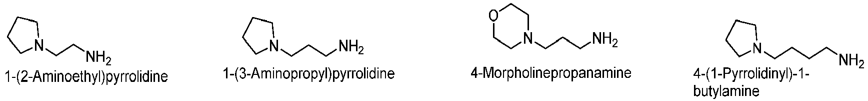

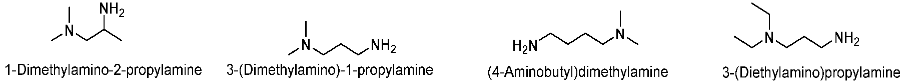

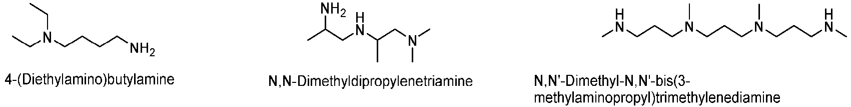

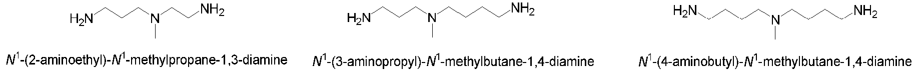

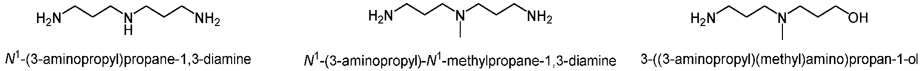

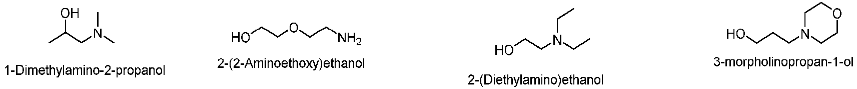

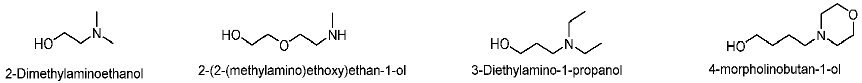

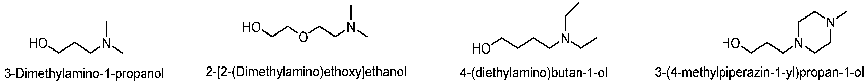

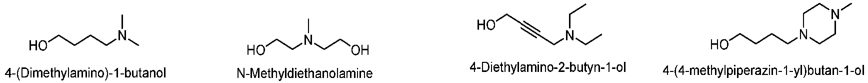

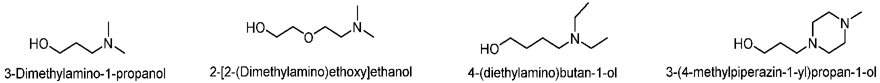

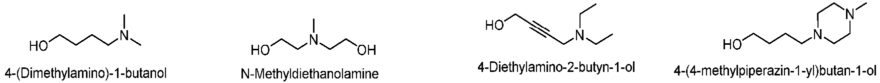

The hydrophobic unit B of Formula I may be introduced by contacting the PE dendron with a functional reagent such as fatty acid or its derivatives. The fatty acid may be saturated or unsaturated fatty acid having C₄-C₂₈ chains. The fatty acid may be, but is not limited to, arachidonic acid, oleic acid, eicosapentanoic acid, lauric acid, caprylic acid, capric acid, myristic acid, palmitic acid, stearic acid, linoleic acid, or linolenic acid. The fatty acid derivative may be, but is not limited to, 12-hydroxy-9-cis-octadecenoic acid, 12-methyltetradecanoic acid, 12-methyltridecanoic acid, 14-methylhexadecanoic acid, 14-methylhexadecanoic acid, 18-methylnonadecanoic acid, 19-methylarachidic acid, isopalmitic acid, isostearic acid, phytanic acid, (±)-2-hydroxyoctanoic acid, (±)-3-hydroxydecanoic acid, (±)-3-hydroxyoctanoic acid, 10-hydroxydecanoic acid, 12-hydroxyoctadecanoic acid, 15-hydroxypentadecanoic acid, 16-hydroxyhexadecanoic acid, 2-hydroxyhexadecanoic acid, 2-hydroxytetradecanoic acid, 2-hydroxydodecanoic acid, DL-α-hydroxystearic acid, DL-β-hydroxylauric acid, DL-β-hydroxymyristic acid, or DL-β-hydroxypalmitic acid.

In an embodiment, the nucleic acid carrier may comprise functional groups suitable for tracking the delivery material *in vitro* and *in* vivo. The nucleic acid carrier may have the fatty acids containing stable isotopes of carbon (C) or hydrogen (H), such as ¹³C or ²H (also referred to herein as deuterium, D or d). When the nucleic acid carrier is formulated into nanoparticles with nucleic acids, such as replicon RNA, the nanoparticles may be tracked *in vitro* and *in vivo* post-administration by techniques such as mass spectroscopy or nuclear magnetic resonance imaging. The inclusion of the stable isotopes may be beneficial for identification of the delivery molecules since these isotopes differ from the abundant in tissues ¹²C and ¹H isotopes. Tracking may be useful for identifying biodistribution, material clearance and molecular stability of nanoparticles post-administration, and related issues. The isotopically labeled fatty acids may be, but are not limited to, octanoic acid-1-¹³C, octanoic acid-8-¹³C, octanoic acid-8,8,8-²H3, octanoic-²H15 acid, decanoic acid-1-¹³C, decanoic acid-10-¹³C, decanoic-10,10,10-²H3 acid, decanoic-²H19 acid, undecanoic acid-1-¹³C, lauric acid-12,12,12-²H3, lauric-²H23 acid, lauric acid-1-¹³C, lauric acid-1,12-¹³C2, tridecanoic-2,2-²H2 acid, myristic acid-14-¹³C, myristic acid-1-¹³C, myristic acid-14,14,14-²H3, myristic-d27 acid, palmitic acid-1-¹³C, palmitic acid-16-¹³C, palmitic acid-16-¹³C,16,16,16-²H3, palmitic acid-²H31, stearic acid-1-¹³C, stearic acid-18-¹³C, stearic acid-18,18,18-²H3, stearic-²H35 acid, oleic acid-1-¹³C, oleic acid-²H34, linolenic acid-1-¹³C, linoleic acid-²H32, arachidonic-5,6,8,9,11,12,14,15-²H8 acid, or eicosanoic-²H39 acid.

In an embodiment, a nanoparticle composition comprising any one of the nucleic acid carriers described herein is provided. A nanoparticle composition herein may be useful to introduce an agent into a cell. The agent may be a nucleic acid. A nanoparticle composition herein may be useful as a vaccine. A nanoparticle composition herein may be useful as a therapeutic formulation. A nanoparticle composition herein may be useful as a transfection agent. A nanoparticle composition herein may be useful in a method of treating or preventing a disease.

In an embodiment, a nanoparticle composition may comprise a mixture of nucleic acid carriers. One or more of the nucleic acid carriers in a mixture may comprise at least one of different amine(s), different amine density(ies), or a different side chain(s) compared to other nucleic acid carriers in the mixture. These nucleic acid carriers may be at a fixed ratio in the mixture. For an example of mixture with three dendritic molecules, a ratio of the first nucleic acid carrier to the second nucleic acid carrier and the third nucleic acid carrier may be i:j:k where i, j and k are independently selected from 1, 2, 3, 4, 5, 6, 7, 8, 9, 10, 11, 12, 13, 14, 15, 16, 17, 18, 19, or 20, or a value between any two of the foregoing.

In an embodiment, the nanoparticle composition may comprise one or more therapeutic or immunogenic nucleic acid agents. As used herein, the term "nucleic acid" refers to any natural or synthetic DNA or RNA molecules, or hybrids thereof. A nucleic acid may comprise natural and/or synthetic nucleotides. The nucleic acid molecule may encode a protein that has therapeutic benefit such as an antibody, hormone, or receptor. The nucleic acid molecule may encode a protein immunogen derived from a disease such as a viral or bacterial pathogen, or from a cancerous cell. The nucleic acid molecule may not encode a protein and instead exert a biological effect through the modulation of cellular signaling or protein expression, for example in the form of siRNA.

In an embodiment, the therapeutic or immunogenic nucleic acid agent may be an RNA or DNA molecule. The nucleic acid agent may also be a mixture of one or more different RNA molecules, DNA molecules, or combination of the two. The term "DNA" or "DNA molecule" or "deoxyribonucleic acid molecule" refers to a polymer of deoxyribonucleotides. The DNA molecule may be a polynucleotide, oligonucleotide, DNA, or cDNA. The DNA molecule may encode wild-type or engineered proteins, peptides or polypeptides, such as antigens. The term "RNA" or "RNA molecule" or "ribonucleic acid molecule" refers to a polymer of ribonucleotides (e.g., 2, 3, 4, 5, 10, 15, 20, 25, 30, or more ribonucleotides). The RNA molecule may be a replicon RNA (repRNA), small interfering RNA (siRNA), miRNA, single strand guide RNA (sgRNA), messenger RNA (mRNA), or transfer RNA (tRNA). Replicon RNA (repRNA) refers to a replication-competent, progeny-defective RNA virus genome that is incapable of producing infectious progeny virions. Viral genomes that are typically modified for use as repRNAs include "positive strand" RNA viruses. The modified viral genomes function as both mRNA and templates for replication. Small interfering RNA (siRNA) refers to an RNA (or RNA analog) comprising between about 10-50 nucleotides (or nucleotide analogs) which is capable of directing or mediating RNA interference. MicroRNAs (miRNAs) refers to small (20-24 nt) regulatory non-coding RNAs that are involved in post-transcriptional regulation of gene expression in eukaryotes by affecting either or both the stability and translation of coding mRNAs. Messenger RNAs (mRNAs) are usually single-stranded RNAs and define the amino acid sequence of one or more polypeptide chains. This information is translated during protein synthesis when ribosomes bind to the mRNA. The DNA or RNA molecules may be chemically modified in nucleic acid backbone, the ribose sugar moiety and the nucleobase itself.

The RNA molecule may be a monocistronic or polycistronic mRNA. A monocistronic mRNA refers to an mRNA comprising only one sequence encoding a protein, polypeptide or peptide. A polycistronic mRNA typically refers to two or more sequences encoding two or more proteins, polypeptides or peptides. An mRNA may encode a protein, polypeptide, or peptide that acts as an antigen.

In an embodiment, the DNA molecule may be a polynucleotide, oligonucleotide, DNA, or cDNA. The RNA molecule may be a replicon RNA (repRNA), small interfering RNA (siRNA), miRNA, single strand guide RNA (sgRNA), messenger RNA (mRNA), or transfer RNA (tRNA). The therapeutic or immunogenic nucleic acid agent may be non-covalently bound or covalently bound to the nucleic acid carrier. The therapeutic or immunogenic nucleic acid agent may be electrostatically bound to the charged nucleic acid carrier through an ionic bond.

In an embodiment, the nanoparticle compositions described herein may include immunogenic or therapeutic nucleic acid agents encoding antigens.

As used herein, "encapsulated" can refer to a nanoparticle that provides an active agent or therapeutic agent, such as a nucleic acid (*e.g.,* a messenger RNA), with full-encapsulation, partial encapsulation, or both. In a preferred embodiment, the nucleic acid is fully encapsulated in the nanoparticle. In the context of nucleic acid therapeutic agents, full encapsulation may be determined by a Ribogreen^{®} assay. RiboGreen^{®} is an ultra-sensitive fluorescent nucleic acid stain for quantitating oligonucleotides and single-stranded DNA or RNA in solution (available from Thermo Fisher Scientific - US)

"Antigen" as used herein is defined as a molecule that triggers an immune response. The immune response may involve either antibody production, or the activation of specific immunologically active cells, or both. The antigen may refer to any molecule capable of stimulating an immune response, including macromolecules such as proteins, peptides, or polypeptides. The antigen may be a structural component of a pathogen, or a cancer cell. The antigen may be synthesized, produced recombinantly in a host, or may be derived from a biological sample, including but not limited to a tissue sample, cell, or a biological fluid.

The antigen may be but is not limited to a vaccine antigen, parasite antigen, bacterial antigen, tumor antigen, environmental antigen, therapeutic antigen or an allergen. As used herein a nucleotide vaccine is a DNA- or RNA-based prophylactic or therapeutic composition capable of stimulating an adaptive immune response in the body of a subject by delivering antigen(s). The immune response induced by vaccination typically results in development of immunological memory, and the ability of the organism to quickly respond to subsequent encounter with the antigen or infectious agent.

The use of a "nucleic acid carrier" herein as a carrier of nucleic acids is preferred and the name "nucleic acid carrier" is applied for that reason. However, embodiments also include the combination of a nucleic acid carrier herein with an agent that contains negative or partially negative charges. The agent may be a drug.

In an embodiment, the nanoparticle composition may be formulated to include drugs that contain negative or partially negative charges. The nanoparticles may be formulated via the electrostatic association of the negative charge with the positive charge of protonated amine groups in the nucleic acid carrier. The negatively charged drugs may be ionic drugs. The term "ionic drug" refers to an electrically asymmetric molecule, which is water soluble and ionizable in solution of distilled water. The ionic drugs may contain phosphate, phosphonate, or phosphinate functional groups. The drugs including phosphate groups may be phosphate-containing nucleotide analogs, for example, drugs used for treating cancer and viral chemotherapy. The phosphate-containing drugs may be, but are not limited to, purine and pyrimidine nucleoside analogs, Arabinosylcytosine (ara-C), Ara-C monophosphate (ara-CMP), azidothymidine (AZT), AZT monophosphate (AZTMP), 2'3'-dideoxycytidine (ddCD), cyclic adenoside monophosphate (cAMP), tenofovir, or adefovir.

In an embodiment, the nanoparticle composition described herein may comprise one or more "lipid conjugate". The lipid conjugate may be useful to prevent the aggregation of particles. Lipid conjugates contemplated include, but are not limited to, PEG-lipid conjugates. PEG-lipid conjugates may be but are not limited to PEG coupled to lipids (e.g., DMG-PEG 2000 or ALC 0159), PEG coupled to phospholipids (e.g., phosphatidylethanolamine (PEG-PE)), PEG conjugated to cholesterol or a derivative thereof, and mixtures thereof. In certain instances, the PEG may be optionally substituted by an alkyl, alkoxy, acyl, or aryl group.

Phosphatidylethanolamines having a variety of acyl chain groups of varying chain lengths and degrees of saturation can be conjugated to PEG to form the lipid conjugate. Phosphatidylethanolamines are commercially available, or can be isolated or synthesized using conventional techniques. The phosphatidylethanolamines may comprise saturated or unsaturated fatty acids with carbon chain lengths in the range of C₁₀ to C₂₀. The phosphatidylethanolamines may comprise mono- or polyunsaturated fatty acids and mixtures of saturated and unsaturated fatty acids. The phosphatidylethanolamines contemplated include, but are not limited to, dimyristoylphosphatidylethanolamine (DMPE), dipalmitoyl-phosphatidylethanol amine (DPPE), dioleoylphosphatidylethanolamine (DOPE), and distearoyl-phosphatidylethanolamine (DSPE).

The PEG-lipid may comprise PEG conjugated to cholesterol or cholesterol derivative. Examples of cholesterol derivatives include, but are not limited to, cholestanol, cholestanone, cholestenone, coprostanol, cholesteryl-2'-hydroxyethyl ether, cholesteryl-4'-hydroxybutyl ether, and mixtures thereof.

PEG is a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Avanti Polar Lipids. The PEG moiety of the PEG-lipid conjugates described herein may comprise an average molecular weight ranging from about 550 daltons to about 10,000 daltons.

The size, relative quantity and distribution of the PEG-lipid, included in the nanoparticle composition may affect physical properties of the nanoparticle composition. The physical properties that can be controlled may be, but are not limited to, diameter of the nanoparticle, the propensity of the nanoparticles to aggregate, the number of nucleic acid molecules inside each nanoparticle, or the concentration of the nanoparticles in the nanoparticle composition, the efficacy of the intra-cellular delivery of therapeutic and immunogenic nucleic acid agents, and/or the efficacy of uptake of the nanoparticles by cells. See a)PCT/US19/67402 (Poulami Talukder, Jasdave S. Chahal, Justine S. McPartlan, Omar Khan, Karl Ruping. Nanoparticle Compositions for Efficient Nucleic Acid Delivery and Methods of Making and Using the Same; b) Mui, Barbara L et al. "Influence of Polyethylene Glycol Lipid Desorption Rates on Pharmacokinetics and Pharmacodynamics of siRNA Lipid Nanoparticles." Molecular therapy. Nucleic acids vol. 2,12 e139. 17 Dec. 2013, doi:10.1038/mtna.2013.66) which are incorporated herein by reference as if fully set forth.

The nanoparticle composition may comprise 10 mol% or less of the PEG-lipid per nanoparticle composition. The nanoparticle composition may comprise about 10 mol %, about 9 mol %, about 8 mol %, about 7 mol %, about 6 mol %, about 5 mol %, about 4 mol %, about 3 mol %, about 2 mol %, or about 1 mol %, or any amount in between any two of the foregoing integers of the PEG-lipid per nanoparticle composition. The nanoparticle composition comprising the PEG-lipid may comprise nanoparticles with a smaller diameter than nanoparticles of the composition lacking the PEG-lipid. The nanoparticle composition may also comprise nanoparticles having a higher propensity of the nanoparticles to aggregate than nanoparticles of the composition lacking the PEG-lipid.

The nanoparticle composition may comprise an "amphipathic lipid." As used herein, "amphipathic lipid" refers to any material having non-polar hydrophobic "tails" and polar "heads." Polar groups may include phosphate, carboxylic, sulfato, amino, sulfhydryl, nitro, hydroxyl, or other like groups. Nonpolar groups may include, but are not limited to, long-chain saturated and unsaturated aliphatic hydrocarbon groups and such groups substituted by one or more cycloalkyl, cycloalkenyl, aryl, heteroaryl, or heterocycle group(s). Examples of amphipathic lipids include, but are not limited to, phospholipids, aminolipids, and sphingolipids. Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidyl choline, dioleoylphos- phatidylcholine, distearoylphosphatidylcholine, and dilinoleoylphosphatidylcholine.

The nanoparticle composition may comprise the amphipathic lipid in the amount ranging from 10 mol% to 15 mol% of the amphipathic lipid per nanoparticle composition.

In an embodiment, the nanoparticle composition may comprise cholesterol or cholesterol derivative. Examples of cholesterol derivatives include, but are not limited to, cholestanol, 5,6-epoxy cholestanol, cholestanone, cholestenone, coprostanol, cholesteryl-2'-hydroxyethyl ether, cholesteryl-4'-hydroxybutyl ether, 24-ethyl cholesterol, 24-methyl cholesterol, cholenic Acid, 3-hydroxy-5-cholestenoic Acid, cholesteryl palmitate, cholesteryl arachidonate, cholesteryl arachidate, cholesteryl myristate, cholesteryl palmitoleate, cholesteryl lignocerate, cholesteryl oleate, cholesteryl stearate, cholesteryl erucate, cholesterol α-linolenate, cholesteryl linoleate, cholesteryl homo-γ-linolenate, 4-hydroxy cholesterol, 6-hydroxy cholesterol, 7-hydroxy cholesterol, 19-hydroxy cholesterol, 20-hydroxy cholesterol, 22-hydroxy cholesterol, 24-hydroxy cholesterol, 25-hydroxy cholesterol, 27-hydroxy cholesterol, 27-alkyne cholesterol, 7-keto cholesterol, 7-dehydro cholesterol, 8-dehydro cholesterol, 24-dehydro cholesterol, 5α-hydroxy-6-keto cholesterol, 20, 22-dihydroxy cholesterol, 7,25-dihydroxy cholesterol, 7,27-dihydroxy cholesterol, 7-keto-25-hydroxy cholesterol, fucosterol, phytosterol, cholesteryl 11, 14-eicosadienoate, dimethyl hydroxyethyl aminopropane carbamoyl cholesterol iodide and mixtures thereof. The cholesterol derivative may comprise a sugar moiety such as mannose, galactose. The cholesterol derivative may comprise a sugar moiety and/or amino acids such as serine, threonine, lysine, histidine, arginine or their derivatives. The nanoparticle composition may comprise the cholesterol or cholesterol derivative in an amount ranging from 50 mol% to 75 mol% of cholesterol or derivative thereof per nanoparticle composition.

In an embodiment, the nanoparticle composition may include a pharmaceutically acceptable carrier. As used herein, the term "pharmaceutically-acceptable carrier" means a pharmaceutically-acceptable material, composition or vehicle, for example a liquid or solid filler, diluent, excipient, manufacturing aid (e.g., lubricant, talc magnesium, calcium or zinc stearate, or stearic acid), or solvent encapsulating material, involved in carrying or transporting the subject compound from one organ, or portion of the body, to another organ, or portion of the body. Each pharmaceutically-acceptable carrier is "acceptable" in the sense of being compatible with the other ingredients of the formulation and not injurious to the patient. Some examples of materials which may serve as pharmaceutically-acceptable carriers include: (1) sugars, for example lactose, glucose, mannose and/or sucrose; (2) starches, for example corn starch and/or potato starch; (3) cellulose, and its derivatives, for example sodium carboxymethyl cellulose, methylcellulose, ethyl cellulose, microcrystalline cellulose and/or cellulose acetate; (4) powdered tragacanth; (5) malt; (6) gelatin; (7) lubricating agents, for example magnesium stearate, sodium lauryl sulfate and/or talc; (S) excipients, for example cocoa butter and/or suppository waxes; (9) oils, for example peanut oil, cottonseed oil, safflower oil, sesame oil, olive oil, corn oil and/or soybean oil; (10) glycols, for example propylene glycol; (11) polyols, for example glycerin, sorbitol, and/or mannitol; (12) esters, for example glycerides, ethyl oleate and/or ethyl laurate; (13) agar; (14) buffering agents, for example magnesium hydroxide and/or aluminum hydroxide; (15) alginic acid; (16) pyrogen-free water; (17) diluents, for example isotonic saline, and/or PEG400; (I8) Ringer's solution; (19) C2-C12 alcohols, for example ethanol; (20) fatty acids; (21) pH buffered solutions; (22) bulking agents, for example polypeptides and/or amino acids (23) serum component, for example serum albumin, HDL and LDL; (24) surfactants, for example polysorbates (Tween 80) and/or poloxamers; and/or (25) other non-toxic compatible substances employed in pharmaceutical formulations: for example, fillers, binders, wetting agents, coloring agents, release agents, coating agents, sweetening agents, flavoring agents, perfuming agents, preservatives and/or antioxidants. The terms "excipient," "carrier," "pharmaceutically acceptable carrier." or the like are used interchangeably herein.

An embodiment comprises a method for treating or preventing a disease or condition in a subject. The method may comprise providing any one of the nanoparticle compositions described herein. The method may comprise administering a therapeutically effective amount of the nanoparticle composition to a subject.

As used herein, the term "therapeutically effective amount" refers to the amount of nanoparticle composition which is effective for producing a desired therapeutic effect. The therapeutic effect may be in at least a subpopulation of cells in an animal. The therapeutic effect may be achieved at a reasonable benefit/risk ratio applicable to medical treatment. A "therapeutically effective amount" may refer to an amount sufficient to generate appearance of antigen-specific antibodies in serum. A "therapeutically effective amount" may refer to an amount sufficient to cause a decrease in disease symptoms. A "therapeutically effective amount" may refer to an amount sufficient to cause a disappearance of disease symptoms. When treating viral infection, a decrease of disease symptoms may be assessed by decrease of virus in faeces, in bodily fluids, or in secreted products. The nanoparticle compositions may be administered using an amount and by a route of administration effective for generating an immune response. The nanoparticle compositions may be administered using an amount and by a route of administration effective for modulating an immune response. The nanoparticle composition may be administered to induce immune tolerance, *i.e.,* to reduce a subject's immune responses against an encoded antigen. For example, an embodiment includes a method of treating allergic or autoimmune diseases that may comprise administering a nanoparticle composition to induce immune tolerance.

Therapeutic efficacy may depend on effective amounts of active agents and time of administration necessary to achieve a desired result. Administering a nanoparticle composition may be a preventive measure. Administering of a nanoparticle composition may be a therapeutic measure to promote immunity to the infectious agent, to minimize complications associated with the slow development of immunity especially in patients with a weak immune system, elderly or infants.

The exact dosage may be chosen by the clinician based on a variety of factors and in view of individual patients. Dosage and administration may be adjusted to provide sufficient levels of the active agent or agents or to maintain the desired effect. For example, factors which may be taken into account may include the type and severity of a disease; age and gender of the patient; drug combinations; and an individual response to therapy.

Therapeutic efficacy and toxicity of active agents in a nanoparticle composition may be determined by standard pharmaceutical procedures, for example, by determining the therapeutically effective dose in 50% of the population (ED50) and the lethal dose to 50% of the population (LD50) in cells cultured *in vitro* or experimental animals. Nanoparticle compositions may be evaluated based on the dose ratio of toxic to therapeutic effects (LD50/ED50), called the therapeutic index, the large value of which may be used for assessment. The data obtained from cell and animal studies may be used in formulating a dosage for human use.

The therapeutically effective dose may be estimated initially from cell culture assays. A therapeutically effective dose may be formulated in animal models to achieve a circulating plasma concentration range that includes the IC50 (*i.e.,* the concentration of the therapeutic which achieves a half-maximal inhibition of symptoms) as determined in cell culture. Levels in plasma may be measured, for example, by high performance liquid chromatography. The effects of any particular dosage may be monitored by a suitable bioassay.

The amount of nanoparticle formulation administered will depend upon the particular therapeutic agent (e.g., nucleic acid) used, the disease or disorder being treated, the age, weight, and condition of the patient, and the judgment of the clinician. A therapeutically effective dose may be between 0.001 ng and 50 mg of the therapeutic or immunogenic nucleic acid per kilogram of body weight of the subject. A therapeutically effective dose may be 0.001 ng, 0.002 ng, 0.003 ng, 0.004 ng, 0.005 ng, 0.006 ng, 0.007 ng, 0.008 ng, 0.009 ng, 0.01 ng, 0.02 ng, 0.03 ng, 0.04 ng, 0.05 ng, 0.06 ng, 0.07 ng, 0.08 ng, 0.09 ng, 0.1 ng, 0.2 ng, 0.3 ng, 0.4 ng, 0.5 ng, 0.6 ng, 0.7 ng, 0.8 ng, 0.9 ng, 0.001 µg, 0.002 µg, 0.003 µg, 0.004 µg, 0.005 µg, 0.006 µg, 0.007 µg, 0.008 µg, 0.009 µg, 0.01 µg, 0.02 µg, 0.03 µg, 0.04 µg, 0.05 µg, 0.06 µg, 0.07 µg, 0.08 µg, 0.09 µg, 0.1 µg, 0.2 µg, 0.3 µg, 0.4 µg, 0.5 µg, 0.6 µg, 0.7 µg, 0.8 µg, 0.9 µg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, or 50 mg of the therapeutic or immunogenic nucleic acid per kilogram of body weight of the subject, or a value in a range between any two of the foregoing. The therapeutic and immunogenic nucleic acid may be a combination of different nucleic acids used per treatment dose. The terms "subject" and "individual" are used interchangeably herein, and mean a human or animal. Preferably, the animal is a vertebrate such as a primate, rodent, domestic animal or game animal. Primates include chimpanzees, cynomolgus monkeys, spider monkeys, and macaques, *e.g*., Rhesus. The rodent may be selected from mice, rats, guinea pigs, woodchucks, ferrets, rabbits and hamsters. The domestic or game animals may be selected from cows, horses, pigs, deer, bison, buffalo, feline species, *e.g.,* domestic cat, canine species, *e.g.,* dog, fox, wolf, avian species, *e.g.,* chicken, emu, ostrich, and fish, *e.g.,* trout, catfish and salmon. A patient or subject may be selected from the foregoing or a subset of the foregoing. A patient or subject may be selected from all of the above, but excluding one or more groups or species such as humans, primates or rodents. In an embodiment, the patient or subject may be a mammal, *e.g.*, a primate, *e.g.,* a human. The terms, "patient" and "subject" are used interchangeably herein. The terms, "patient" and "subject" are used interchangeably herein.

Preferably, the subject is a mammal. The mammal may be a human, non-human primate, mouse, rat, dog, cat, swine, horse, or cow, but is not limited to these examples. Mammals other than humans may be subjects that represent animal models of a disease or disorder. In addition, the methods described herein may be directed to treating domesticated animals and/or pets. A subject may be male or female.

As used herein, the terms "administer," "administering," "administration," or the like refer to the placement of a composition into a subject. The administration may be by a method or route which results in at least partial localization of the composition at a desired site such that desired effect is produced. A nanoparticle composition described herein may be administered by any appropriate route known in the art including, but not limited to, oral or parenteral routes, including intravenous, intramuscular, subcutaneous, transdermal, airway (aerosol), pulmonary, nasal, rectal, or topical (including buccal and sublingual) administration.

Exemplary modes of administration include, but are not limited to, injection, infusion, instillation, inhalation, or ingestion. "Injection" includes without limitation, intravenous, intramuscular, intraarterial, intrathecal, intraventricular, intracapsular, intraorbital, intracardiac, intradermal, intraperitoneal, trans tracheal, subcutaneous, subcuticular, intraarticular, sub capsular, subarachnoid, intraspinal, intracerebro spinal, and intrastemal injection and infusion. In an embodiment, the compositions may be administered by intravenous infusion or injection.

The nanoparticle compositions may be used for delivery of therapeutic DNAs or RNAs that modulate gene expression. The therapeutic nucleic acid may be an antisense oligonucleotide (AON) or a double-stranded small interfering RNA (siRNA). Typically, siRNAs are between 21 and 23 nucleotides in length. The siRNAs may comprise a sequence complementary to a sequence contained in an mRNA transcript of a target gene when expressed within the host cell. The antisense oligonucleotide may be a morpholino antisense oligonucleotide. The antisense oligonucleotide may include a sequence complementary to a sequence contained in an mRNA transcript of a target gene. The therapeutic or immunogenic nucleic acid may be an interfering RNA (iRNA) against a specific target gene within a specific target organism. The iRNA may induce sequence-specific silencing of the expression or translation of the target polynucleotide, thereby down-regulating or preventing gene expression. The iRNA may completely inhibit expression of the target gene. The iRNA may reduce the level of expression of the target gene compared to that of an untreated control. The therapeutic or immunogenic nucleic acid may be a micro RNA (miRNA). The miRNA may be a short RNA, *e.g.,* a hairpin RNA (hpRNA). The miRNA may be cleaved into biologically active dsRNA within the target cell by the activity of the endogenous cellular enzymes. The RNA may be a double stranded RNA (dsRNA). The ds RNA may be at least 25 nucleotides in length or may be longer. The dsRNA may contain a sequence that is complementary to the sequence of the target gene or genes.

In an embodiment, the therapeutic or immunogenic nucleic acid may be or may encode an agent that totally or partially reduces, inhibits, interferes with or modulates the activity or synthesis of one or more genes encoding target proteins. The target genes may be any genes included in the genome of a host organism. The sequence of the therapeutic or immunogenic nucleic acid may not be 100% complementary or identical to the nucleic acid sequence of the target gene.

In an embodiment, the nanoparticle composition may be used for targeted, specific alteration of the genetic information in a subject. An embodiment comprises targeted, specific alteration of the genetic information sufficient to have a therapeutic or immunogenic impact, in a subject comprising administration of a nanoparticle composition herein. As used herein, the term "alteration" refers to any change in the genome in the cells of a subject. The alteration may be insertion or deletion of nucleotides in the sequence of a target gene. "Insertion" refers to addition of one or more nucleotides to a sequence of a target gene. The term "deletion" refers to a loss or removal of one or more nucleotides in the sequence of a target gene. The alteration may be correction of the sequence of a target gene. "Correction" refers to alteration of one or more nucleotides in the sequence of a target gene, *e.g.*, by insertion, deletion or substitution, which may result in a more favorable expression of the gene manifested by improvements in genotype and/or phenotype of the host organism.

The alteration of the genetic information may be achieved via the genome editing techniques. As used herein, "genome editing" refers to the process of modifying the nucleotide sequence in the genome in a precise or controlled manner.

An exemplary genome editing system is a Clustered Regularly Interspaced Short Palindromic Repeats (CRISPR) system as described, for example, in WO 2018/154387, which published August 30, 2018 and is incorporated herein by reference as if fully set forth. In general, "CRISPR system" refers to transcripts and other elements involved in the expression of CRISPR-associated (Cas) genes, including sequences encoding a Cas gene, a tracr (trans-activating CRISPR) sequence, a tracr-mate sequence, a guide sequence, or other sequences and transcripts from a CRISPR locus. One or more tracr mate sequences may be operably linked to a guide sequence before processing or crRNA after processing by a nuclease. The tracrRNA and crRNA may be linked and may form a chimeric crRNA-tracrRNA hybrid where a mature crRNA is fused to a partial tracrRNA via a synthetic stem loop to mimic the natural crRNA:tracrRNA duplex as described in Cong et al., Science, 15:339(6121):819-823 (2013) and Jinek et al., Science, 337(6096):816-21 (2012), which are incorporated herein by reference as if fully set forth. A single fused crRNA-tracrRNA construct is also referred herein as a guide RNA or gRNA, or single-guide RNA (sgRNA). Within an sgRNA, the crRNA portion is identified as the "target sequence" and the tracrRNA is often referred to as the "scaffold." In an embodiment, the nanoparticle compositions described herein may be used to deliver an sgRNA.

In an embodiment, the nanoparticle compositions may be used to apply other exemplary genome editing systems including meganucleases, homing endonucleases, TALEN-based systems, or Zinc Finger Nucleases. The nanoparticle compositions may be used to deliver the nucleic acid (RNA and/or DNA) that encodes the sequences for these gene editing tools, and the actual gene products, proteins, or other molecules.

In an embodiment, the nanoparticle composition may be used for gene targeting in a subject *in* vivo or *ex vivo, e.g.,* by isolating cells from the subject, editing genes, and implanting the edited cells back into the subject. An embodiment comprises a method comprising administering a nanoparticle composition herein to isolated cells from a subject. The method may include gene targeting. The method may comprise implanting the edited cells back into the subject.

An embodiment comprises a method for introducing an agent into a cell. The method may comprise exposing the cell to a nanoparticle composition herein. The agent may be a nucleic acid. The method may be a method of transfection when the agent is a nucleic acid.

Embodiments List - The following list is a non-limiting list of embodiments and does not limit embodiments otherwise described or exemplified herein. An embodiment in this list may be supplemented with one or more element from any other embodiment or example herein. An element in an embodiment in this list may be replaced by one or more elements from any other embodiment or example herein.
1. A nucleic acid carrier comprising a dendron or dendrimer of Formula Ia, Ib, Ic, Id, Ie or If: 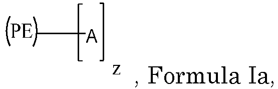 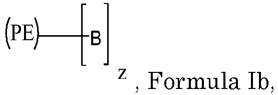 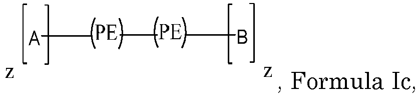 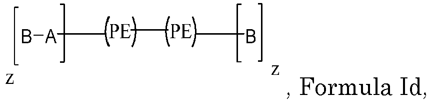 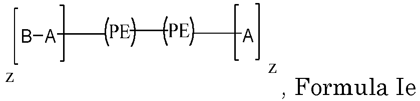 or 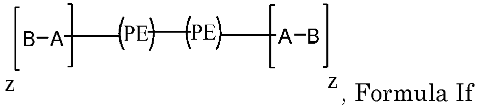 wherein PE is a polyester dendron which includes a core and a plurality of monomeric polyester units that form one or more generations, A is an amine, B is a hydrophobic unit, and z is the number of surface groups.
2. The nucleic acid carrier of embodiment 1, wherein PE has the Formula II:

   [(core)-Gn-O], II,

   wherein G is a layer or generation of dendron and n is a generation number and is in a range from 1 to 10.
3. The nucleic acid carrier of embodiments 1 or 2, wherein the plurality of monomeric polyester units comprise 2,2-bis(hydroxymethyl) propionic acid or 2,2-bis(hydroxymethyl)butyric acid, preferably where all of the plurality of monomeric polyester units are either 2,2-bis(hydroxymethyl) propionic acid or 2,2-bis(hydroxymethyl)butyric acid.
4. The nucleic acid carrier of any one of the previous embodiments, wherein z has Formula III:

   z = bⁿ, III,

   wherein b is branch point multiplicity, or number of branches at each branching point, and n is a generation number and is in a range from 1 to 10.
5. The nucleic acid carrier of any one of the previous embodiments, wherein A is 1-(2-Aminoethyl)piperazine, 1-Piperidineethanamine, 1-Dimethylamino-2-propylamine, 1-(3-Aminopropyl)pyrrolidine, 1-(2-Aminoethyl)pyrrolidine, 1-(2-Aminoethyl)piperidine, 2-(1-Piperazinyl)ethylamine, 2-(4-Methyl-piperazin-1-yl)-ethylamine, 3-(Dimethylamino)-1-propylamine, 3-(Diethylamino)propylamine, (4-Aminobutyl)dimethylamine, 4-(1-Pyrrolidinyl)-1-butylamine, 4-Morpholineethanamine, 4-Morpholinepropanamine, 4-Methyl-1-piperazineethanamine, 4-(Diethylamino)butylamine, N,N-Dimethylethylenediamine, N,N-Dimethyldipropylenetriamine, N,N'-Dimethyl-N,N'-bis(3-methylaminopropyl)trimethylenediamine, N1-(3-aminopropyl)propane-1,3-diamine, N1-(3-aminopropyl)-N1-methylpropane-1,3-diamine, 3-((3-aminopropyl)(methyl)amino)propan-1-ol, 1-Dimethylamino-2-propanol, 1-[Bis[3-(dimethylamino)propyl]amino]-2-propanol, 2-Dimethylaminoethanol, 2-(Diethylamino)ethanol, 2-[2-(Dimethylamino)ethoxy]ethanol, 2-(2-Aminoethoxy)ethanol, 2-{[2-(Dimethylamino)ethyl]methylamino}ethanol, 3-Dimethylamino-1-propanol, 3-Diethylamino-1-propanol, 4-(Dimethylamino)-1-butanol, 4-Diethylamino-2-butyn-1-ol, N-Methyldiethanolamine, 4-(diethylamino)butan-1-ol, 3-(azetidin-1-yl)propan-1-ol, 3-(pyrrolidin-1-yl)propan-1-ol, 3-(piperidin-1-yl)propan-1-ol, 4-(azetidin-1-yl)butan-1-ol, 4-(pyrrolidin-1-yl)butan-1-ol, 4-(piperidin-1-yl)butan-1-ol, 3-morpholinopropan-1-ol, 4-morpholinobutan-1-ol, 3-(4-methylpiperazin-1-yl)propan-1-ol, or 4-(4-methylpiperazin-1-yl)butan-1-ol.
6. The nucleic acid carrier of any one of the previous embodiments, wherein B is derived from a fatty acid (C4-C28) or derivative thereof.
7. The nucleic acid carrier of embodiment 6, wherein the fatty acid is selected from the group consisting of: caprylic acid, capric acid, lauric acid, myristic acid, palmitic acid, stearic acid, oleic acid, linoleic acid, linolenic acid, arachidonic acid, and eicosapentanoic acid.
8. The nucleic acid carrier of embodiment 6, wherein the fatty acid derivative is selected from the group consisting of: 12-hydroxy-9-cis-octadecenoic acid, 12-methyltetradecanoic acid, 12-methyltridecanoic acid, 14-methylhexadecanoic acid, 14-methylhexadecanoic acid, 18-methylnonadecanoic acid, 19-methylarachidic acid, isopalmitic acid, isostearic acid, phytanic acid, (±)-2-hydroxyoctanoic acid, (±)-3-hydroxydecanoic acid, (±)-3-hydroxyoctanoic acid, 10-hydroxydecanoic acid, 12-hydroxyoctadecanoic acid, 15-hydroxypentadecanoic acid, 16-hydroxyhexadecanoic acid, 2-hydroxyhexadecanoic acid, 2-hydroxytetradecanoic acid, 2-hydroxydodecanoic acid, DL-α-hydroxystearic acid, DL-β-hydroxylauric acid, DL-β-hydroxymyristic acid, and DL-β-hydroxypalmitic acid.
9. The nucleic acid carrier of any one of embodiments 6-8, wherein the fatty acid or derivative comprises one or more stable isotopes.
10. The nucleic acid carrier of embodiment 9, wherein the stable isotope is a stable isotope of carbon or hydrogen.
11. The nucleic acid carrier of embodiment 10, wherein the stable isotope of carbon is ¹³C.
12. The nucleic acid carrier of embodiment 10, wherein the stable isotope of hydrogen is ²H.
13. The nucleic acid carrier of embodiment 10, wherein the fatty acid that comprises the stable isotope is selected from the group consisting of: octanoic acid-1-13C, octanoic acid-8-13C, octanoic acid-8,8,8-d3, octanoic-2H15 acid, decanoic acid-1-13C, decanoic acid-10-13C, decanoic-10,10,10-d3 acid, decanoic-d19 acid, undecanoic acid-1-13C, lauric acid-12,12,12-2H3, lauric-2H23 acid, lauric acid-1-13C, lauric acid-1,12-13C2, tridecanoic-2,2-2H2 acid, myristic acid-14-13C, myristic acid-1-13C, myristic acid-14,14,14-2H3, myristic-2H27 acid, palmitic acid-1-13C, palmitic acid-16-13C, palmitic acid-16-13C,16,16,16-2H3, palmitic acid-2H31, stearic acid-1-13C, stearic acid-18-13C, stearic acid-18,18,18-2H3, stearic-2H35 acid, oleic acid-1-13C, oleic acid-2H34, linolenic acid-1-13C, linoleic acid-2H32, arachidonic-5,6,8,9,11,12,14,15-2H8 acid, and eicosanoic-2H39 acid.
14. A nanoparticle composition comprising the nucleic acid carrier of any one of embodiments 1-13, and a therapeutic or immunogenic nucleic acid agent fully or partially encapsulated therein.
15. The nanoparticle composition of embodiment 14, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.
16. The nanoparticle composition of embodiment 14, wherein the therapeutic or immunogenic nucleic acid agent encodes one or more antigens selected from the group consisting of infectious disease, pathogen, cancer, autoimmunity disease and allergenic disease.
17. The nanoparticle composition of embodiment 14, wherein the therapeutic or immunogenic nucleic acid agent comprises an RNA or DNA capable of silencing, inhibiting or modifying the activity of a gene.
18. The nanoparticle composition of any one of embodiments 14-17 further comprising a PEG-lipid.
19. The nanoparticle composition of embodiment 18, wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000.
20. The nanoparticle composition of embodiment 18 or 19, wherein the nanoparticle composition comprises the PEG-lipid in a range from 1 mol% to 10 mol% of the PEG-lipid per nanoparticle composition.
21. The nanoparticle composition of any one of embodiments 18-20 further comprising a phospholipid and cholesterol or derivative thereof.
22. The nanoparticle composition of claim 21, wherein the phospholipid is 1,2-dioleoyl-sn-glycero-3-phosphoethanolamine (DOPE) or distearoylphosphatidylcholine (DSPC).
23. The nanoparticle composition of embodiment 21 or 22, wherein the nanoparticle composition comprises the phospholipid in a range from 10 mol% to 15 mol% of the phospholipid per nanoparticle composition.
24. The nanoparticle composition of any one of embodiments 21-23, wherein the nanoparticle composition comprises the cholesterol or derivative thereof in a range from 50 mol% to 75 mol% of the cholesterol or derivative thereof per nanoparticle composition.
25. A method for treating or preventing a disease or condition in a subject comprising: administering a therapeutically effective amount of the nanoparticle composition of any one of embodiments 1-24 to a subject.
26. The method of embodiment 25, wherein the therapeutically effective amount of the nanoparticle composition comprises the therapeutic or immunogenic nucleic acid agent in a range from 0.01 mg nucleic acid to 10 mg nucleic acid per kg body weight of the subject.
27. The method of embodiment 25 or 26, wherein the subject is a mammal.
28. The method of any one of embodiments 25-27, wherein the mammal is selected from the group consisting of: a rodent, a canine, a primate, an equine, a high value agricultural animal, and a human.

### EXAMPLES

The following non-limiting examples are provided to illustrate particular embodiments. The embodiments throughout may be supplemented with one or more details from one or more examples below, and/or one or more elements from an embodiment may be substituted with one or more details from one or more examples below.

An ideal nucleic acid carrier could be biodegradable to prevent bioaccumulation and subsequent cytotoxicity. High generation have higher packaging and thus difficult to biodegrade. There is a need for low generation dendrons that can complex with nucleic acids and translocate across cellular membranes while maintaining biodegradability and avoiding cytotoxicity.

### Example 1. Nucleic Acid Carrier of Formula [I]a

In an embodiment, the chemical bonds present on the monomeric unit are ester bonds and the terminal layer of low generation polyester dendrons were substituted with amines through amide bonds. This develops a structure-activity relationship understanding of how surfactant-like dendrons with amine surface groups and a lipophilic unit at their focal points can self-assemble and subsequently bind to nucleic acids with high affinity.

Scheme 1 is a schematic drawing of the generation 2 modified polyester dendron and amines (A) that can be used for modification. In this scheme, the alkyl chain B could be selected from any one of C₁₀ - C₂₈. An example of the synthesis of PE-Dendron-G2-Hexadecyl-propylpyrrolidine as follows.

### Synthetic Scheme1:

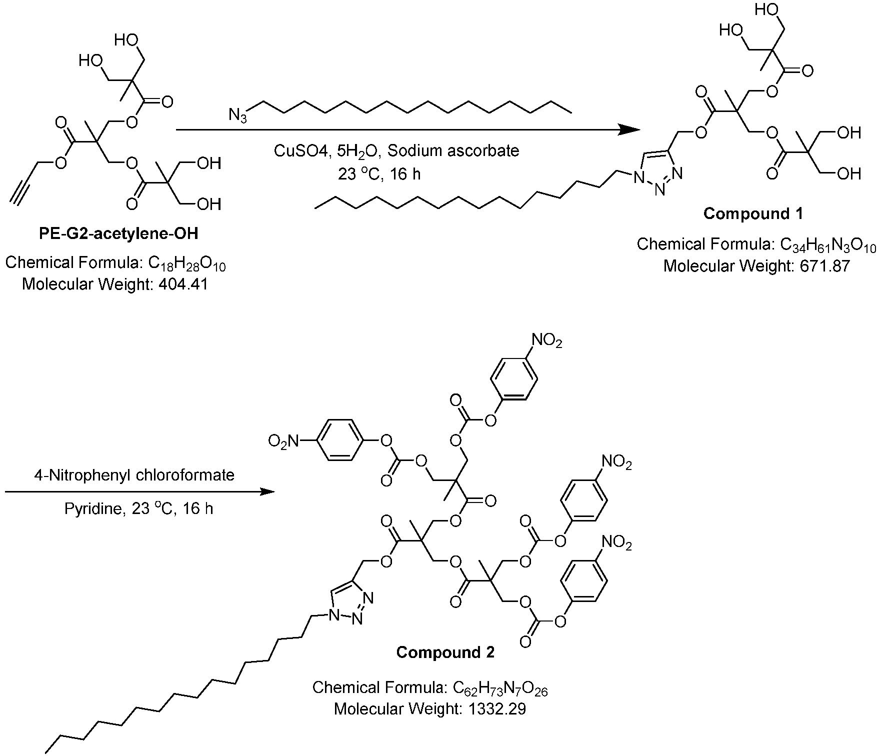

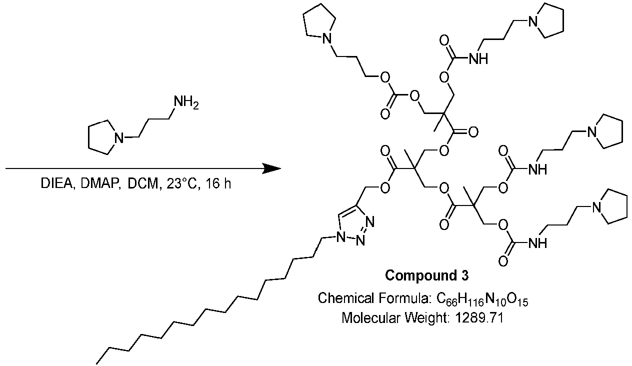

**Compound 1:** Hexadecyl Azide (MW: 267.2, 106 mg, 0.39 mmol) was dissolved in 1 ml THF, taken in 50 ml round bottom flask (RBF), then Alkyne, PE-G2-acetylene-OH (80 mg, 0.2 mol) dissolved in THF (0.5 mL) was added along with CuSO₄.5H₂O (7.8 mg, 0.031mmol, 10 mol%) and sodium ascorbate (12.3 mg, 0.062 mmol, 20 mol%), and degassed THF: H₂O (2 mL, 1:1), The reaction mixture was stirred 16 h at 23°C. Next day the solvent was then removed in vacuo, purified via flash chromatography on silica column (24 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 65% mobile phase b. (*R*_{f} = 0.6 in 1:1 mobile phase a/ mobile phase b) to yield the desired product as yellow oil with yield 120 mg (90%).

**Compound 2:** Compound 1 (120 mg, 0.18 mmol) was dissolved in dry DCM or CH₂Cl₂ (3 mL) and pyridine (0.3 ml, 1.44 mmol) was added followed by p-nitrophenyl chloroformate (580 mg, 2.88 mmol, MW 201.6, 16 eq) dissolved in dry DCM (5 mL). Next day the reaction mixture was diluted with 1.33 M NaHSO₄ and extracted with DCM. The organic layer was washed with brine, concentrated in Rotavap and purified by flash chromatography with DCM/EtOAc. The compound eluted at 8.5% EtOAc/DCM, (*R*_{f} = 0.7 in 9:1 DCM/EtOAc). The yield of the reaction 175 mg (74%). 1H NMR (301 MHz, CHLOROFORM-d) δ ppm 0.83 - 0.89 (m, 3 H) 1.20 - 1.35 (m, 40 H) 1.79 - 1.90 (m, 2 H) 2.02 - 2.04 (m, 3 H) 4.06 - 4.15 (m, 2 H) 4.26 - 4.45 (m, 13 H) 4.45 - 4.49 (m, 1 H) 5.25 - 5.30 (m, 2 H) 7.33 - 7.41 (m, 8 H) 7.57 - 7.61 (m, 1 H) 8.18 - 8.30 (m, 8 H).

¹³C NMR (76 MHz, CHLOROFORM-d) δ ppm 14.09 (s, 1 C) 14.17 (s, 1 C) 17.58 (s, 1 C) 17.62 (s, 1 C) 21.03 (s, 1 C) 22.65 (s, 1 C) 26.45 (s, 1 C) 28.95 (s, 1 C) 29.31 (s, 1 C) 29.36 (s, 1 C) 29.49 (s, 1 C) 29.56 (s, 1 C) 29.61 (s, 1 C) 29.64 (s, 1 C) 30.22 (s, 1 C) 31.88 (s, 1 C) 46.53 (s, 1 C) 46.67 (s, 1 C) 50.44 (s, 1 C) 58.42 (s, 1 C) 60.37 (s, 1 C) 65.70 (s, 1 C) 69.08 (s, 1 C) 77.20 (s, 1 C) 121.75 (s, 1 C) 123.91 (s, 1 C) 125.26 (s, 1 C) 125.28 (s, 1 C) 125.30 (s, 1 C) 141.61 (s, 1 C) 145.54 (s, 1 C) 152.07 (s, 1 C) 155.17 (s, 1 C) 170.97 (s, 1 C) 172.01 (s, 1 C).

**Compound 3:** A solution of Compound 2 (90 mg, 0.07 mmol) dissolved in dry DCM (0.9 mL) was added to an excess of amine, 1-(3-Aminopropyl)pyrrolidine (72mg, 0.56 mmol) dissolved in dry DCM (1 mL). A solution of DMAP (17 mg, 0.28 mmol) and DIPEA (0.05 mmol, 0.56 mmol) in dry DCM (1 mL) was added and the reaction mixture was stirred 16 h at 23°C under an argon atmosphere. Next day the solvent was then removed in vacuo, purified via flash chromatography on silica column (24 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 80% mobile phase b. (*R*_{f} = 0.2 (75:22:3 CH₂Cl₂/MeOH/NH₄OHaq) to yield the desired product as yellow oil (30 mg, 35%). MS (ESI) calcd for C₆₆H₁₁₆N₁₀O₁₅ [M + H]+ m/z 1289.7, found 1289.7.

### Example 2. Nucleic Acid Carrier of Formula [I]b

In the present application, the chemical bonds present on the monomeric unit are ester bonds and the terminal layer of low generation polyester dendrons with amine on the focal point were substituted with hydrophobic tails through ester bonds making the whole structure prone to hydrolysis and thus biodegradable. Such low generation dendrons with alkyl chains can be noncovalently combined with nucleic acids to form nanoparticles through their dynamic equilibrating nature. This develops a structure-activity relationship understanding of how surfactant-like dendrons with hydrophobic unit surface groups (B) and amine (A) at their focal points can self-assemble and subsequently bind to nucleic acids with high affinity. Representative structure of this example is shown below.

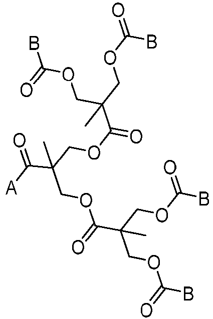

### Example 3. Nucleic Acid Carrier of Formula [I]c:

The structure comprises two, covalently attached, dendritic segments, which may be of different generation, and linear chains are attached to one of the dendritic segments. The synthetic strategy utilized the chemoselectivity of the Copper-catalyzed azide-alkyne cycloadditions (CuAAC) reaction. Two separate dendrons with azide and primary acetylene in the focal points were efficiently coupled in THF, where one dendron has amines in the periphery and the other has hydrophobic tails in the periphery. Representative structure of this example is shown below. 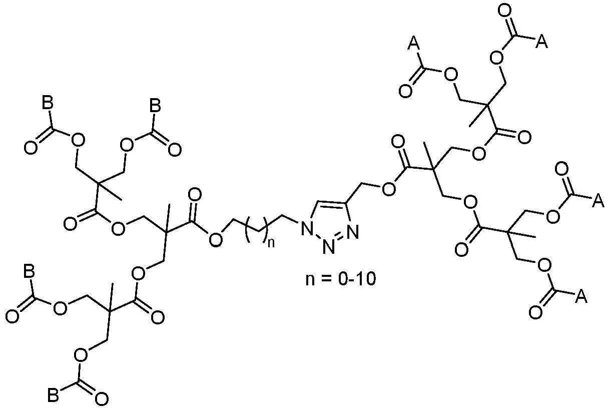 or

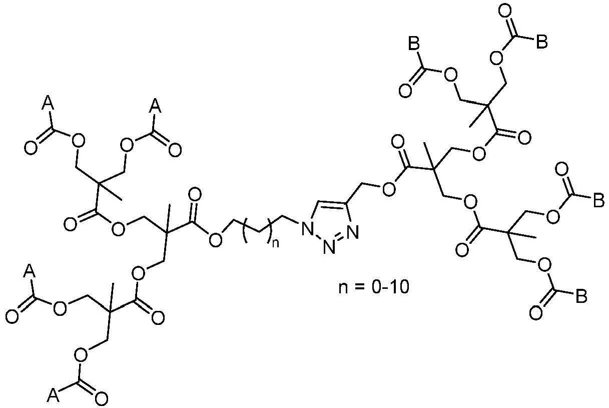

### Synthetic Scheme 2:

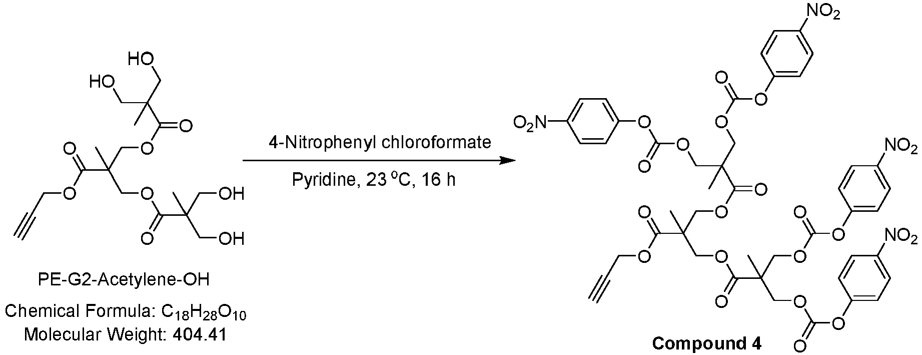

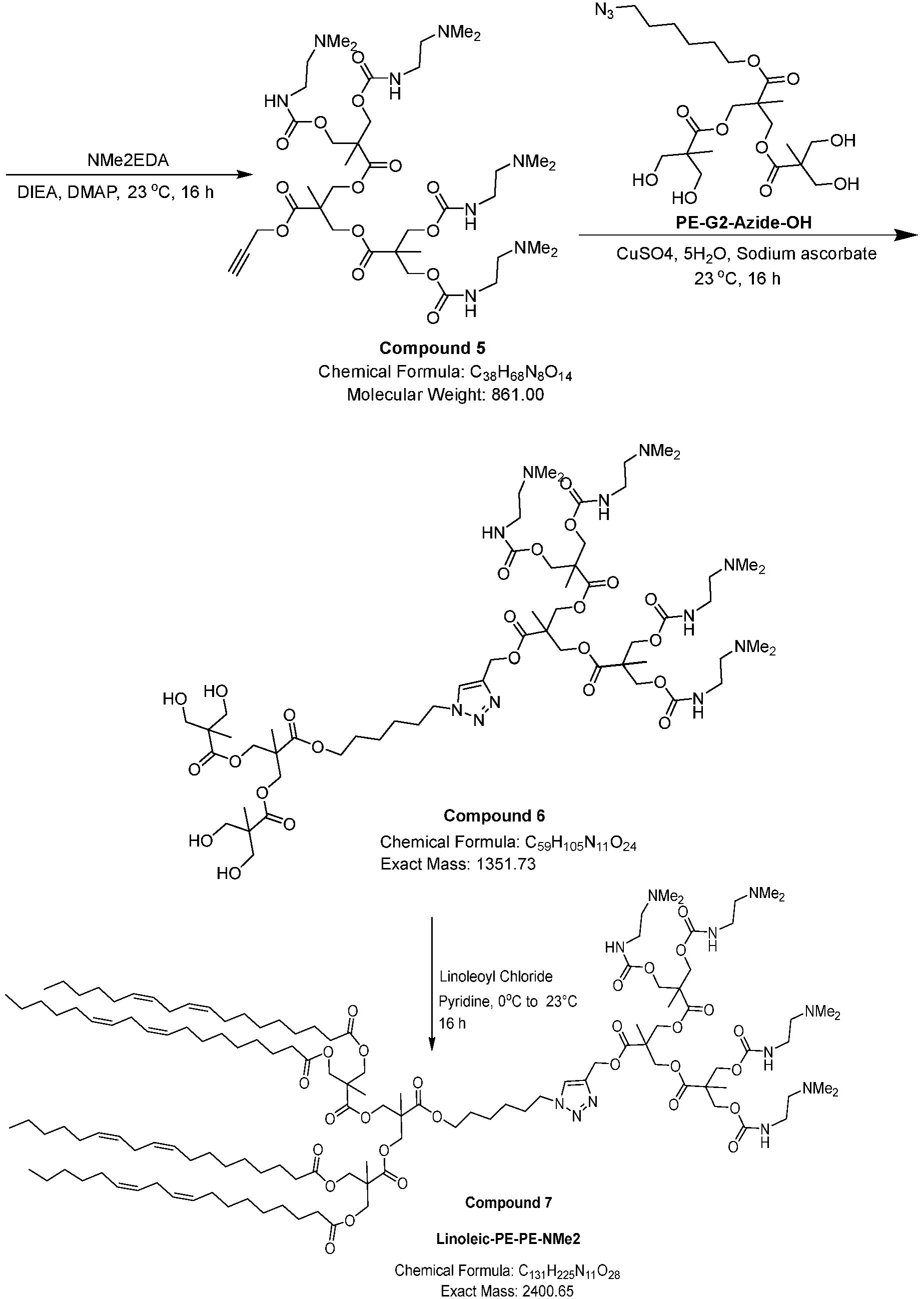

**Compound 4:** Starting material, PE Dendron G2 acetylene-OH, (300 mg, 0.62 mmol) was dissolved in dry DCM (6 mL) and pyridine (0.9 ml, 4.96 mmol) was added followed by p-nitrophenyl chloroformate (2.1 g, 10 mmol) dissolved in dry DCM (15 mL), the reaction mixture was stirred at 0°C to rt for 16 h, Next day TLC shows product formation. The reaction mixture was diluted with 1.33 M NaHSO₄, extracted with EtOAc. The organic layer was washed with brine, dried over Na₂SO₄ and evaporated in Rotavap. The crude was then purified by flash chromatography with DCM/EtOAc. The compound started eluted at 45% EtOAc (*R*_{f} = 0.9 in 1:9 EtOAc/DCM) to yield the desired product as light yellow oil (553 mg, 70%).

**Compound 5:** A solution of compound 4 (150 mg, 0.14 mmol) dissolved in dry DCM (3 mL) was added to an excess of NMe₂EDA (0.08 ml, 0.7 mmol) dissolved in dry DCM (1 mL). A solution of DMAP (34 mg, 0.28 mmol) and DIPEA (0.1 ml, 0.56 mmol) in dry DCM (1 mL) was added and the reaction mixture was stirred 16 h at 23°C under a nitrogen atmosphere. The solvent was then removed in vacuo, loaded onto 24 g column, eluted at 60% ultra/DCM. (silica gel TLC *R*_{f} = 0.25 in 75:22:3 CH₂Cl₂/MeOH/NH4OHaq; Yield 120 mg (90%). MS (ESI) calcd for C₃₈H₆₈N₈O₁₄ [M]+ m/z 861.00, found 861.4.

**Compound 6:** PE-G2-azide-OH (MW:491.54, 54 mg, 114 µmol) was taken in 50 ml RBF, then Alkyne (MW:860, 98 mg, 114 µmol) dissolved in THF (0.6 mL) was added along with CuSO₄.5H₂O (3 mg, 11.4 µmol, 10 mol%, MW 249.69) and sodium ascorbate (4.5 mg, 22.8 µmol, 20 mol%, MW 198.11), and degassed THF: H₂O (2 mL, 1:1), The reaction mixture was stirred 16 h at 23°C. Next day the solvent was then removed in vacuo, purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 70% mobile phase b. (silica gel TLC *R*_{f} = 0.2 in mobile phase b; Yield 120 mg (90%). MS (ESI) calcd for C₅₉H₁₀₅N₁₁O₂₄ [M+H]+ m/z 1352.73, found 1352.6.

**Compound 7:** Compound 6 (30 mg, 0.022 mmol) was dissolved into pyridine (2 ml). After 10 minutes flushing with argon in ice bath, Linoleoyl chloride (0.04 ml, 0.11 mmol, 5.5 eq, MW 298, SD 0.93) was added dropwise, stirred at 0°C to 23°C for 16 h. Next day the solvent was then removed in vacuo, purified via flash chromatography on silica column (24 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 90% mobile phase b. (silica gel TLC *R*_{f} = 0.45 in mobile phase b); Yield 20 mg (36%). 1H NMR (301 MHz, CHLOROFORM-d) δ ppm 0.82 - 0.90 (m, 12 H) 1.12 - 1.16 (m, 6 H) 1.19 - 1.39 (m, 79 H) 1.50 - 1.67 (m, 9 H) 1.97 - 2.07 (m, 16 H) 2.16 - 2.24 (m, 24 H) 2.24 - 2.31 (m, 9 H) 2.37 - 2.45 (m, 6 H) 2.71 - 2.79 (m, 7 H) 3.18 - 3.28 (m, 6 H) 4.01 - 4.29 (m, 24 H) 4.32 - 4.39 (m, 1 H) 5.24 - 5.40 (m, 16 H) 5.59 - 5.70 (m, 2 H) 7.71 - 7.76 (m, 1 H). MS (ESI) calcd for C₁₃₁H₂₂₅N₁₁O₂₈ [M+2H]2+ m/z 1201.3, found 1201.8.

### Synthetic Scheme 3:

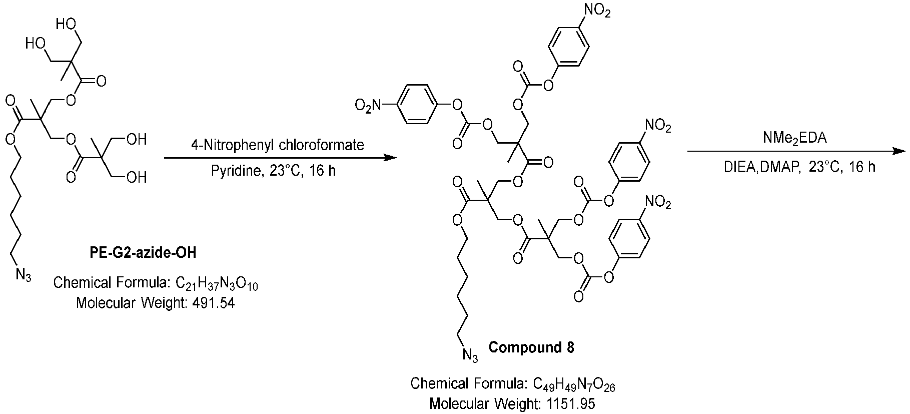

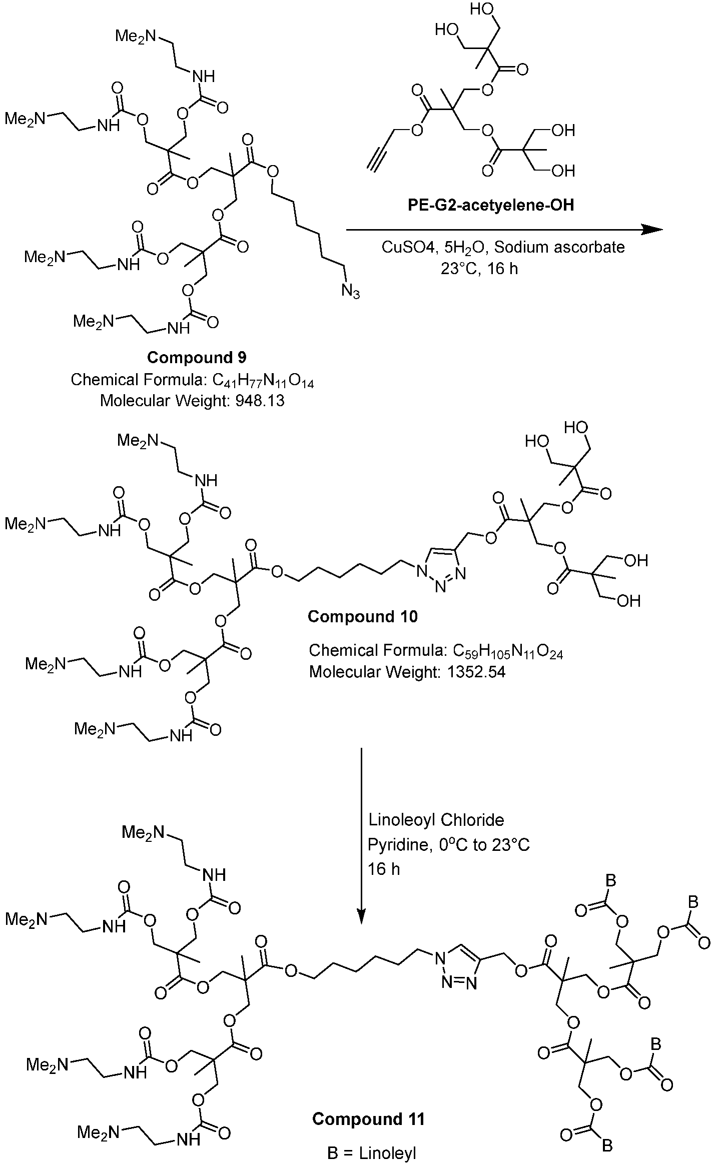

**Compound 8:** PE-G2-azide-OH (250 mg, 0.51 mmol) was dissolved in dry DCM (6 mL) and pyridine (0.9 ml, 11.2 mmol) was added followed by p-nitrophenyl chloroformate (1.6 g, 8.16 mmol, MW 201.6, 16 eq) dissolved in dry DCM (16 mL), the reaction mixture was stirred at 0°C to 23°C for 16 h, Next day TLC shows product formation. The reaction mixture was diluted with 1.33 M NaHSO4, extracted with EtOAc. The organic layer was washed with brine, dried over Na₂SO₄ and evaporated in Rotavap. The crude was then purified by flash chromatography with DCM/EtOAc. The compound eluted at 4% EtOAc/DCM, (*R*_{f} = 0.8 (19:1 DCM/EtOAc). Yield 135 mg (23%).

**Compound 9:** A solution of Compound 8 (135 mg, 0.117 mmol) dissolved in dry DCM (1 mL) was added to an excess of NMe2EDA (0.077 ml, 0.7 mmol) dissolved in dry DCM (1 mL). A solution of DMAP (29 mg, 0.23 mmol) and DIPEA (0.08 ml , 0.46 mmol) in dry DCM (1 mL) was added and the reaction mixture was stirred 16 h at 23°C under an argon atmosphere. Next day the solvent was then removed in vacuo, purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 60% mobile phase b. (silica gel TLC *R*_{f} = 0.35 in mobile phase b). Yield 70 mg (63%).

**Compound 10:** Compound 9 (70 mg, 0.074 mmol) was taken in 50 ml RBF, Alkyne (29.9 mg, 0.074 mol) dissolved in 1ml THF was added to azide, CuSO₄.5H₂O (2 mg, 0.0074 mmol, 10 mol%) was added along with sodium ascorbate (3 mg, 0.015 mmol, 20 mol%), and degassed THF: H₂O (2 mL, 1:1), The reaction mixture was stirred 16 h at 23°C. Next day the solvent was then removed in vacuo, purified via flash chromatography on silica column (24 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 70% mobile phase b. (silica gel TLC *R*_{f} = 0.25 in mobile phase b). Yield 50 mg (50%). MS (ESI) calcd for C₅₉H₁₀₅N₁₁O₂₄ [M+H]+ m/z 1352.73, found 1352.6.

**Compound 11:** Compound 10 (50 mg, 0.036 mmol) was dissolved into pyridine (2 ml). After 10 minutes flushing with argon in ice bath, Linoleoyl chloride (0.065 ml, 0.203 mmol, 5.5 eq, MW 298, SD 0.93) was added dropwise, stirred at 0°C to 23°C for 16 h. Next day the solvent was then removed in vacuo, purified via flash chromatography on silica column (40 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 70% mobile phase b. (silica gel TLC *R*_{f} = 0.85 in mobile phase b); Yield 30 mg (34%). 1H NMR (301 MHz, CHLOROFORM-d) δ ppm 0.82 - 0.91 (m, 12 H) 1.14 - 1.41 (m, 84 H) 1.50 - 1.68 (m, 10 H) 1.96 - 2.07 (m, 15 H) 2.17 - 2.25 (m, 26 H) 2.26 - 2.31 (m, 6 H) 2.36 - 2.45 (m, 7 H) 2.70 - 2.79 (m, 7 H) 3.17 - 3.27 (m, 6 H) 4.07 - 4.24 (m, 22 H) 4.27 - 4.38 (m, 3 H) 5.22 - 5.41 (m, 16 H) 5.57 - 5.66 (m, 2 H) 7.65 - 7.69 (m, 1 H). MS (ESI) calcd for C₁₃₁H₂₂₅N₁₁O₂₈ [M+2H]2+ m/z 1201.3, found 1201.8.

### Example 4. Nucleic Acid Carrier of Formula [I]d:

The structure comprises two, covalently attached, dendritic segments, which may be of different generation, and linear chains are attached to one of the dendritic segments. The synthetic strategy also utilized the chemoselectivity of the CuAAC reaction. Two separate dendrons with azide and primary acetylene in the focal points were efficiently coupled in THF. Embodiments here allow introduction of different types of tails in the same structure. Representative structure of this example is shown below.

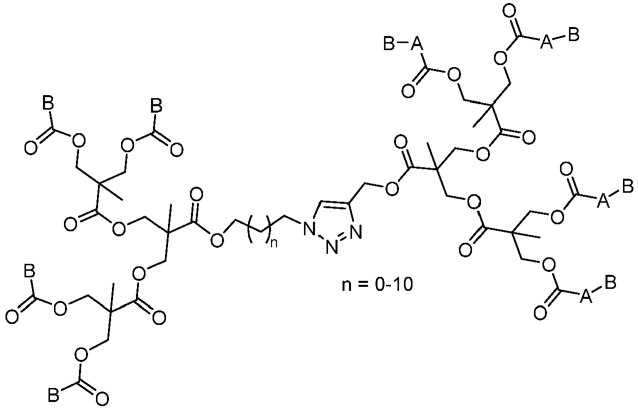

### Synthetic Scheme 4:

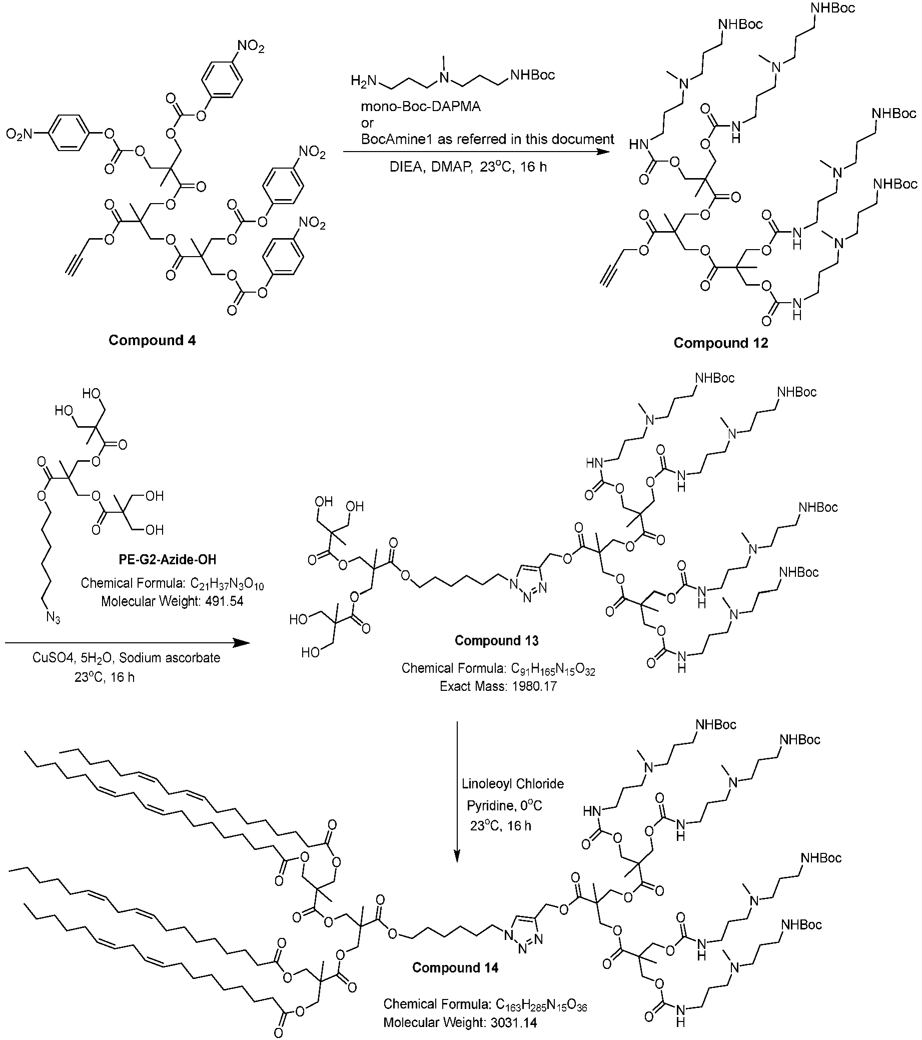

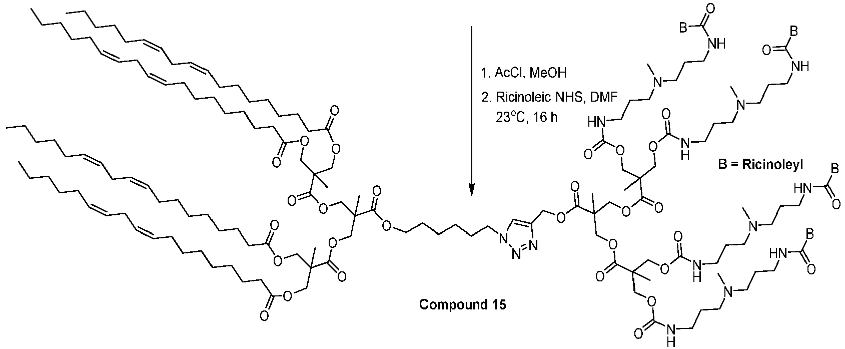

**Compound 12:** A solution of compound 4 (438 mg) dissolved in dry DCM (6 mL) was added to an excess of mono-Boc-DAPMA (0.45 ML) dissolved in dry DCM (6 mL). A solution of DMAP (100 mg) and DIPEA (0.29 ml) in dry DCM (1 mL) was added and the reaction mixture was stirred 16 h at 23°C under an argon atmosphere. TLC confirmed the reaction completed. The crude product was concentrated under reduced pressure in Rotavap, and purified via flash chromatography on silica column with gradient elution from 100% CH₂Cl₂ (mobile phase a) to 75:22:3 CH₂Cl₂/MeOH/NH₄OHaq (by volume, (mobile phase b) over 40 minutes. The desired product eluted at 45% mobile phase b. (*R*_{f} = 0.4 in 1:1 mobile phase a/ mobile phase b) to yield the desired product as light yellow oil (404 mg, 66%).

**Compound 13:** PE-G2-Azide-OH (MW:491.54, 58 mg, 114 µmol) was taken in 50 ml RBF, then compound 12 (MW:1475.82, 173 mg, 114 µmol) dissolved in THF (1 mL) was added along with CuSO₄.5H₂O ( 3 mg, 0.011 mmol, 10 mol%, MW 249.69) and sodium ascorbate (4.6 mg, 0.023 mmol, 20 mol%, MW 198.11), and degassed THF: H₂O (2 mL, 1:1), The reaction mixture was stirred 16 h at 23°C. Next day TLC confirmed the reaction completed. The reaction mixture was purified via flash chromatography on silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 56% mobile phase b. (*R*_{f} = 0.4 (75:22:3 CH₂Cl₂/MeOH/NH₄OHaq) to yield the desired product as yellow oil (140 mg, 60%). MS (ESI) calcd for C₉₁H₁₆₅N₁₅O₃₂ [M + 4H]4+ m/z 795.5, found 794.9; [M + 3H]3+ m/z 1059.96, found 1060.2.

**Compound 14:** Compound 13 (137 mg, 0.069 mmol) was dissolved into pyridine (2 ml). After 10 minutes flushing with argon in ice bath, linoleoyl chloride (0.13 ml, 0.41 mmol, 6 eq, MW 298, SD 0.93) was added dropwise, stirred at 0°C to 23°C for 16 h. The reaction mixture was stirred 16 h at 23°C. Next day TLC confirmed the reaction completed. The reaction mixture was evaporated in Rotavap, purified via flash chromatography on silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 50% mobile phase b. (*R*_{f} = 0.6 (75:22:3 CH₂Cl₂/MeOH/NH₄OHaq) to yield the desired product as yellow oil (100 mg, 48%). MS (ESI) calcd for C₁₆₃H₂₈₅N₁₅O₃₆ [M + 2H]²⁺ m/z 1515.5, found 1516.1; [M + 3H]³⁺ m/z 1010.3, found 1011.0; 1H NMR (301 MHz, CHLOROFORM-d) δ ppm 0.84 - 0.90 (m, 12 H) 1.11 - 1.36 (m, 91 H) 1.40 - 1.43 (m, 40 H) 1.52 - 1.70 (m, 26 H) 1.98 - 2.07 (m, 15 H) 2.16 - 2.29 (m, 20 H) 2.34 - 2.45 (m, 15 H) 2.70 - 2.78 (m, 6 H) 3.07 - 3.25 (m, 17 H) 4.01 - 4.25 (m, 26 H) 4.32 - 4.40 (m, 1 H) 5.27 - 5.43 (m, 19 H) 5.93 - 6.20 (m, 1 H) 7.71 - 7.77 (m, 1 H).

**Compound 15 (PE Dendron-Al-Ricinoleic. PE Dendron-Linoleic):** 100 mg of Compound 14 (0.033 mmol) was treated with 20 eq of AcCl (0.047 ml, 0.66 mmol) after dissolving the compound in 3ml MeOH, the reaction was stirred at 0°C to 23°C for 4 hr, evaporated to dryness and half of the crude product was dissolved in 2 ml DMF, 0.045 ml Et₃N (0.33 mmol) was added followed by Ricinoleic-NHS (39 mg, 0.099 mmol) that was dissolved in 1 ml DMF. Next day he reaction mixture was concentrated and purified via flash chromatography on silica column with gradient elution from 100% CH₂Cl₂ (mobile phase a) to 75:22:3 CH₂Cl₂/MeOH/NH₄OHaq (by volume, (mobile phase b) over 40 minutes. The desired product eluted at 45% mobile phase b. (*R*_{f} = 0.85 (75:22:3 CH₂Cl₂/MeOH/NH₄OHaq) to yield the desired product as yellow oil (50 mg, 41%). ¹H NMR (301 MHz, CHLOROFORM-d) δ ppm 0.84 - 0.89 (m, 22 H) 1.06 - 1.17 (m, 14 H) 1.20 - 1.33 (m, 133 H) 1.41 - 1.46 (m, 13 H) 1.53 - 1.70 (m, 37 H) 1.98 - 2.07 (m, 22 H) 2.11 - 2.21 (m, 34 H) 2.23 - 2.32 (m, 7 H) 2.35 - 2.44 (m, 18 H) 2.71 - 2.78 (m, 4 H) 3.13 - 3.30 (m, 19 H) 3.55 - 3.63 (m, 5 H) 3.66 - 3.78 (m, 1 H) 4.02 - 4.39 (m, 27 H) 5.18 - 5.57 (m, 20 H) 6.17 - 6.28 (m, 2 H) 6.82 - 6.91 (m, 2 H) 7.70 - 7.80 (m, 1 H).

### Example 5. Nucleic Acid Carrier of Formula [I]e:

The structure comprises two, covalently attached, dendritic segments, which may be of different generation, and linear chains being attached to one of the dendritic segments. Two separate dendrons with azide and primary acetylene in the focal points will be coupled to form heterofunctional dendritic structure with one segment will be modified to incorporate amines on the surface whereas the other segment will be modified to incorporate both amines and tails. This invention allows to introduce different types of amines in the same structure where one amine may condense nucleic acid whereas the other amine may have strong buffering capacity inside the endosomes and thus a further influx of hydrochloric acid and water, leading eventually to endosomal rupture of the payload. Representative structure of this example is shown below.

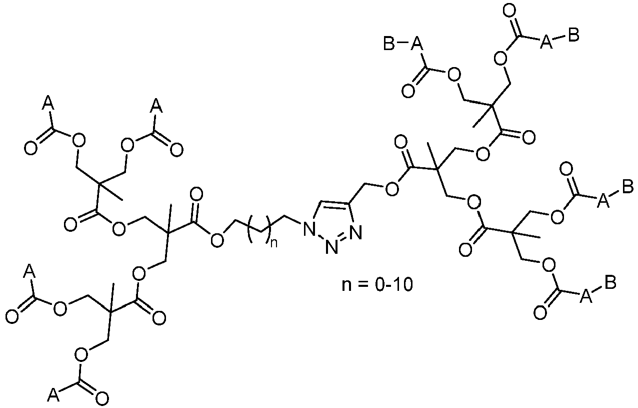

### Synthetic Scheme 5:

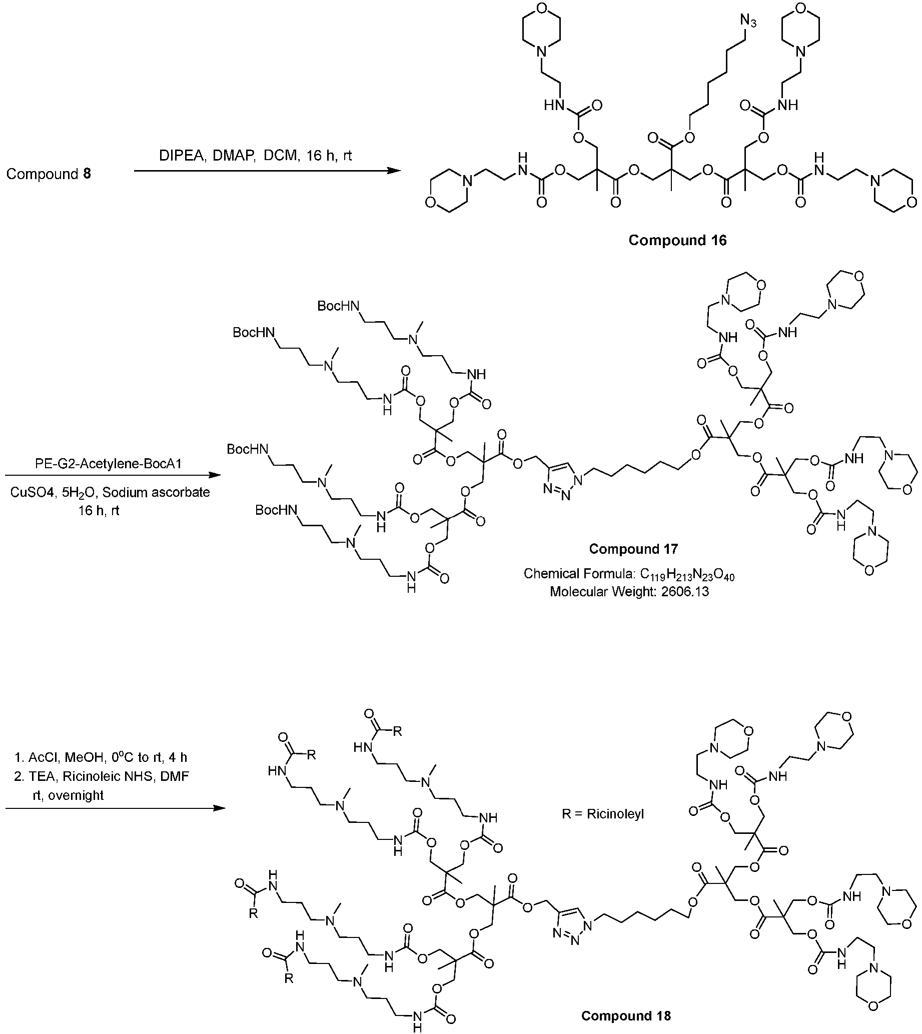

**Compound 16:** A solution of compound 8 (90 mg, MW 1151.95, 0.078 mmol) dissolved in dry DCM (0.5 mL) was added to an excess of 4-(2-aminoethyl)
morpholine (80 mg, 0.992 g/ml, MW 130.19, 0.63 mmol, 8 eq) dissolved in dry DCM (1 mL). A solution of DMAP (19 mg, MW 122.17, 0.156 mmol, 2 eq) and DIPEA (0.312 mmol, MW 129.24, d 0.742 g/ml, 4 eq) in dry DCM (1 mL) was added and the reaction mixture was stirred 16 h at 23°C under an argon atmosphere. TLC confirmed the reaction completed. The crude product was concentrated under reduced pressure in Rotavap, and purified via flash chromatography on 24 g silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 24% mobile phase b. (*R*_{f} = 0.7 in 1:1 mobile phase a/ mobile phase b) to yield the desired product as light yellow oil (79 mg, 91%).

**Compound 17:** Compound 16 (MW:1116, 79 mg, 80 µmol) was taken in 50 ml RBF, then Alkyne, PE-G2-acetylene-BocAmine1 or compound 12 **(MW:1489,** 105 mg, 70 µmol) dissolved in THF (0.6 mL) was added along with CuSO₄.5H₂O (2 mg, 0.07 µmol, 10 mol%, MW 249.69) and sodium ascorbate (3 mg, 14.8 µmol, 20 mol%, MW 198.11), and degassed THF: H₂O (2 mL, 1:1), The reaction mixture was stirred 16 h at 23°C. Next day TLC confirmed the reaction completed. The reaction mixture was purified via flash chromatography on 24g silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 65% mobile phase b. (*R*_{f} = 0.2 (2:1 mobile phase b/ mobile phase a) to yield the desired product as yellow oil (75 mg, 41%). MS (ESI) calcd for C119H213N23O40 [M + 3H]3+ m/z, 869.4 found 869.18. **Compound 18:** 50 mg of Compound 17 (0.019 mmol) was treated with 20 eq of (0.024 ml, 0.38 mmol) after dissolving the compound in 3ml MeOH, the reaction was stirred at 0°C to 23°C for 4 h, evaporated to dryness and the crude product was dissolved in 2 ml DMF, Et₃N (0.053 ml, 0.38 mmol, MW 101.19, d 0.726) was added followed by Ricinoleic-NHS (46 mg, 0.115 mmol, MW 395.27) that was dissolved in 1 ml DMF. Next day the reaction mixture was concentrated and purified via flash chromatography on silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 75% mobile phase b. (*R*_{f} = 0.55 (75:22:3 CH₂Cl₂/MeOH/NH4OHaq)) to yield the desired product as yellow oil (50 mg, 41%).
MS (ESI) calcd for C171H309N23O40 [M + 2H]2+ m/z 1664.6, found 1664.4

### Example 6. Nucleic Acid Carrier of Formula [I]f:

The structure comprises two, covalently attached, dendritic segments, which may be of different generation, and linear chains being attached to one of the dendritic segments. Two separate dendrons with azide and primary acetylene in the focal points respectively will be coupled to form homofunctional dendritic structure Representative structures of this example is shown below.

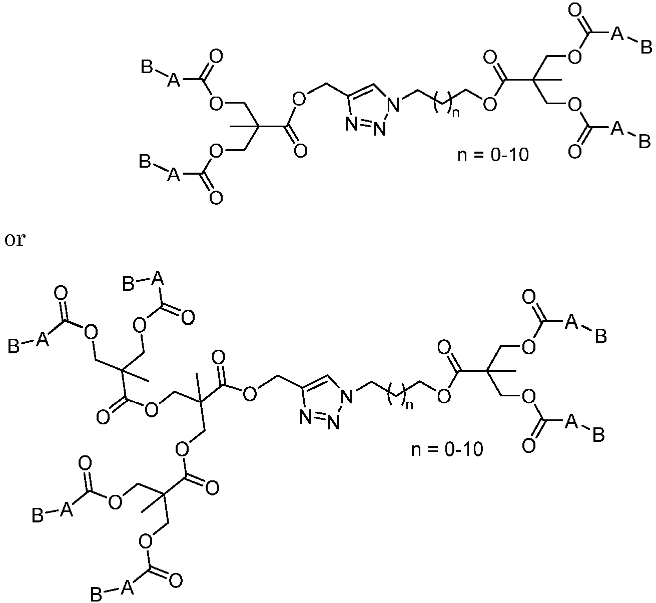

### Synthetic Scheme 6:

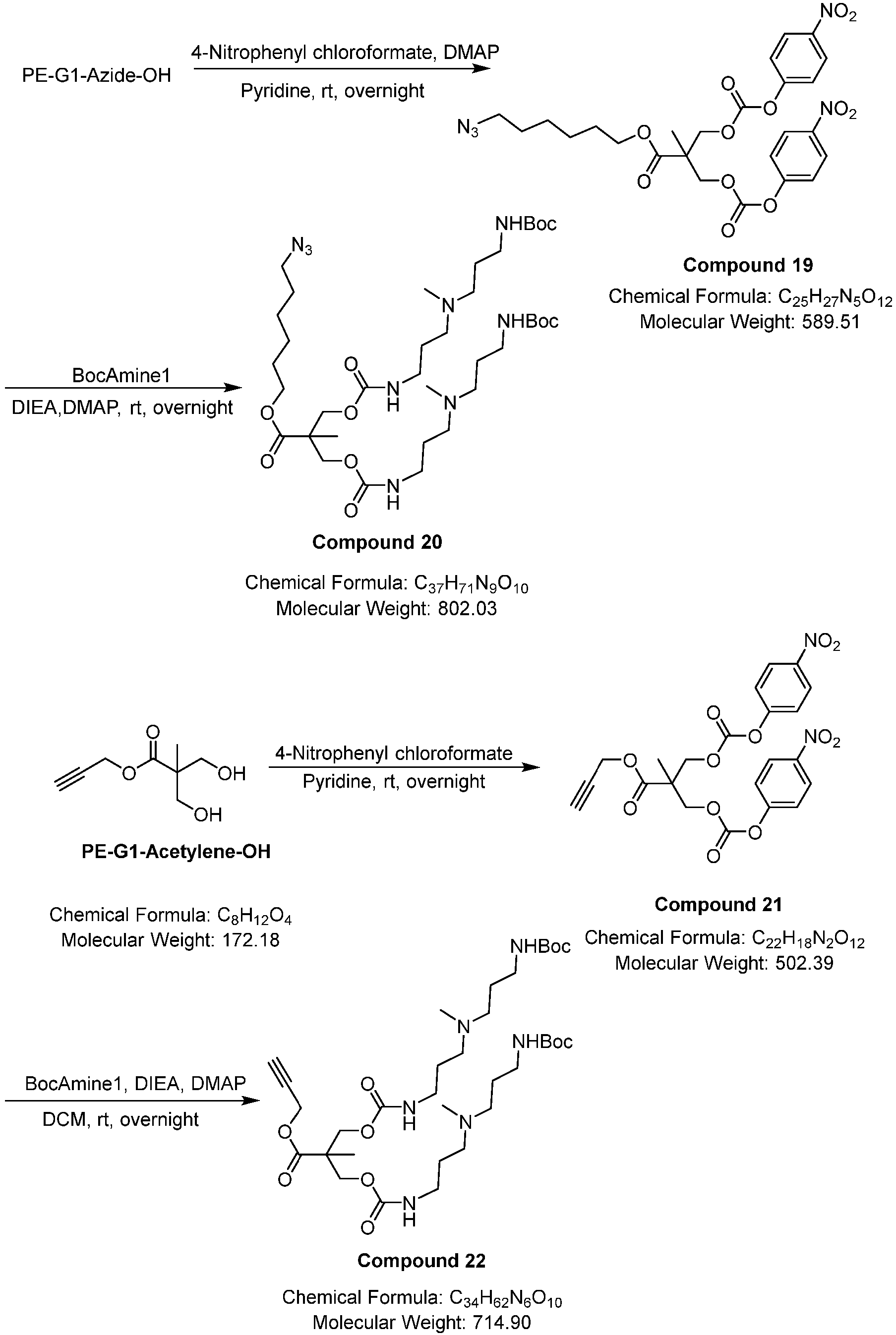

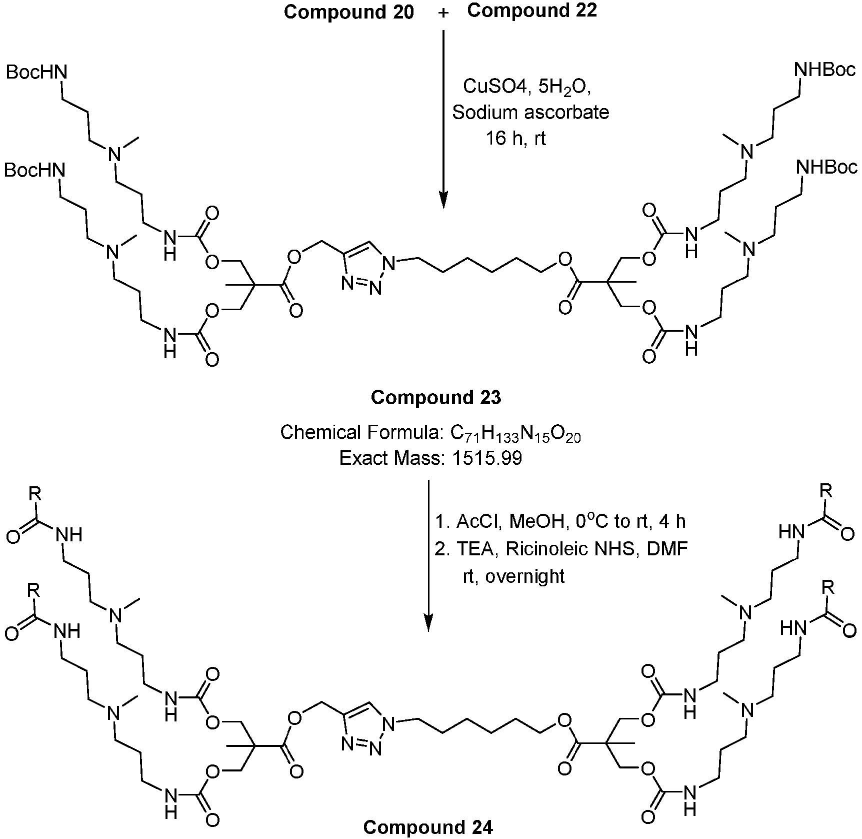

**Compound 19:** PE-G1-azide-OH (250 mg, 0.96 mmol, MW 259.3) was dissolved in dry DCM (3 mL) and pyridine (0.6 ml, 7.68 mmol, 8 eq) was added followed by *p*-nitrophenyl chloroformate (525 mg, mmol, MW 201.6, 2.7 eq) dissolved in dry DCM (8 ml), the reaction mixture was stirred at 0°C to 23°C for 16 h. Next day TLC shows product formation. The reaction mixture was diluted with 1.33 M NaHSO4, extracted with EtOAc. The organic layer was washed with brine, dried over Na₂SO₄ and evaporated in Rotavap. The crude was then purified by flash chromatography with DCM/EtOAc. The compound eluted at 2% EtOAc/DCM, (*R*_{f} = 0.8 (19:1 DCM/EtOAc). Yield 190 mg (33%).

**Compound 20:** A solution of Compound 19 (190 mg, 0.32 mmol, 589.51) dissolved in dry DCM (0.5 mL) was added to an excess of BocAmine1 (237 mg, 0.97 mmol, 3 eq) dissolved in dry DCM (1 mL). A solution of DMAP (79 mg, 0.64 mmol) and DIPEA (0.22 ml, 1.28 mmol, MW 129.24, d 0.742) in dry DCM (1 mL) was added and the reaction mixture was stirred 16 h at 23°C under an argon atmosphere. Next day the solvent was then removed in vacuo, purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 40% mobile phase b. (silica gel TLC *R*_{f} = 0.55 in mobile phase a/mobile phase b). Yield 140 mg (54%).

**Compound 21:** PE-G1-Acetylene-OH (641 mg, 3.73 mmol) was dissolved in dry DCM (6 mL) and (5.4 ml, 30 mmol) was added followed by p-nitrophenyl chloroformate (2 g, 10 mmol, 2.7 eq) dissolved in dry DCM (15 mL), the reaction mixture was stirred at 0°C to 23°C for 16 h, Next day TLC shows product formation. The reaction mixture was diluted with 1.33 M NaHSO4, extracted with EtOAc. The organic layer was washed with brine, dried over Na₂SO₄ and evaporated in Rotavap. The crude was then purified by flash chromatography with DCM/EtOAc. The compound eluted at DCM, (*R*_{f} = 0.7 (19:1 DCM/EtOAc). Yield 765 mg (41%).

**Compound 22:** A solution of Compound 21 (350 mg, 0.70 mmol, MW 502.4) dissolved in dry DCM (3 mL) was added to an excess of BocAmine1 (513 mg, 2.09 mmol, MW 245.4) dissolved in dry DCM (6 mL). A solution of DMAP (170 mg, 1.39 mmol) and DIPEA (0.49 ml , 2.8 mmol) in dry DCM (1 mL) was added and the reaction mixture was stirred 16 h at 23°C under an argon atmosphere. Next day the solvent was then removed in vacuo, purified via flash chromatography on silica column (40 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 50% mobile phase b. (silica gel TLC *R*_{f} = 0.55 in mobile phase a/ mobile phase b). Yield 300 mg (60%).

**Compound 23:** Azide, compound 20 (MW:802.03 , 70 mg, 0.087 mol) was taken in 50 ml RBF, then Alkyne, compound 22 (MW:742.48, 65 mg, 0.087 mmol) dissolved in THF (0.6 mL) was added along with CuSO₄.5H₂O (7 mg, 30 mol%, MW 249.69) and sodium ascorbate ( 11 mg, 60 mol%, MW 198.11), and degassed THF: H₂O (2 mL, 1:1), The reaction mixture was stirred 16 h at 23°C. Next day the solvent was then removed in vacuo, purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 65% mobile phase b. (silica gel TLC *R*_{f} = 0.65 in 1:1 mobile phase a/mobile phase b). Yield 70 mg (50%). MS (ESI) calcd for C71H133N15O20 [M+2H]2+ m/z 759.0, found 758.6.

**Compound 24:** 70 mg of Compound 23 (0.027 mmol) was treated with 20 eq of AcCl (0.04 ml, 0.54 mmol) after dissolving the compound in 3ml MeOH, the reaction was stirred at 0°C to 23°C for 5 hr, evaporated to dryness and the crude product was dissolved in 2 ml DMF, Et₃N (0.08 ml, 0.54 mmol) was added followed by Ricinoleic-NHS (64 mg, 0.16 mmol, 6 eq) that was dissolved in 1 ml DMF. Next day the reaction mixture was concentrated and purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 74% mobile phase b. (*R*_{f} = 0.85 (2:1 mobile phase b/ mobile phase a)) to yield the desired product as yellow oil (32 mg, 31%). MS (ESI) calcd for C₁₂₃H₂₂₉N₁₅O₂₀ [M+2H]2+ m/z 1119.9, found 1119.3; [M+3H]3+ m/z 746.6, found 746.5

### Synthetic Scheme 7:

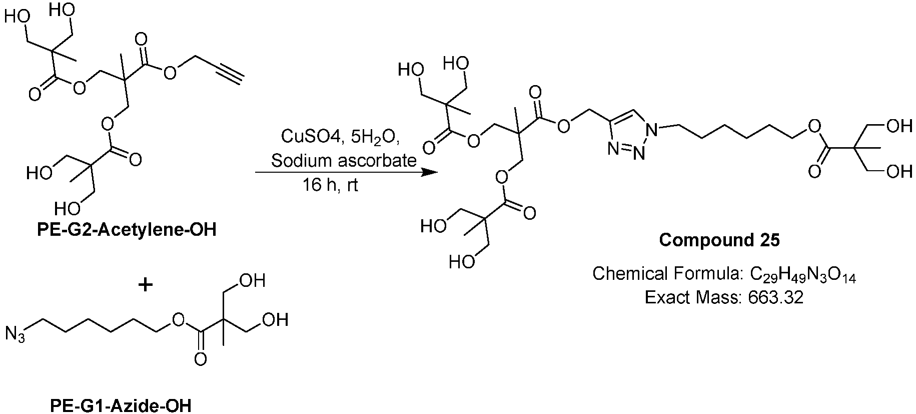

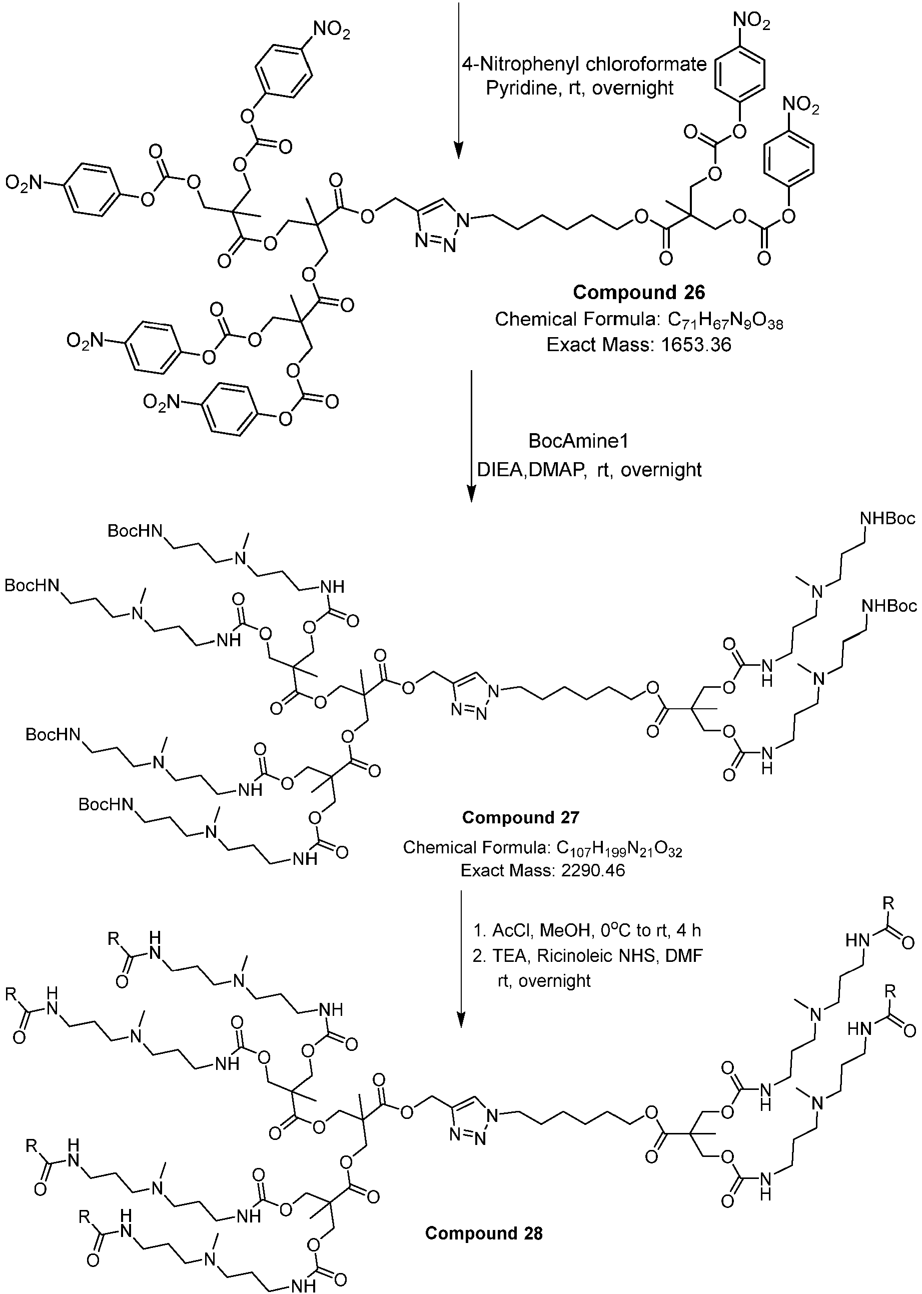

**Compound 25:** Azide, PE-G1-Azide-OH (230 mg, 0.89 mol, MW 259.3 ) was taken in 25 ml RBF, then Alkyne, PE-G2-Acetylene-OH (327 mg, 0.80 mmol, MW 404.4 ) dissolved in THF (0.6 mL) was added along with CuSO₄.5H₂O (22 mg, 10 mol%, MW 249.69, used 35 mg) and sodium ascorbate (35 mg, 20 mol%, MW 198.11: added actual 50 mg), and degassed THF: H₂O (2 mL, 1:1), the reaction mixture was stirred 16 h at 23°C. Next day the solvent was then removed in vacuo, purified via flash chromatography on silica column (24 g) with gradient elution from 100% CH₂Cl₂ (mobile phase a) to 75:22:3 CH₂Cl₂/MeOH/NH₄OHaq (by volume, mobile phase b) over 40 minutes. The desired product eluted at 65% mobile phase b. (silica gel TLC *R*_{f} = 0.60 in 1:1 mobile phase a/mobile phase b). Yield 398 mg (74%, MW 663.32). MS (ESI) calcd for C₂₉H₄₉N₃O₁₄ [M+H]+ m/z 663.3, found 663.8.

**Compound 26:** Compound 25 (349 mg, 0.526 mmol) was dissolved in dry DCM (8.5 mL) and pyridine (0.34 ml, 4.21 mmol, 8 eq) was added to it, cooled in dry ice, p-nitrophenyl chloroformate (1.7g, 8.42 mmol, 201.5 MW) dissolved in dry DCM (10 mL) was added, the reaction mixture was stirred at 0°C to rt for 16 h, Next day TLC shows product formation, washed with 1.33 M NaHSO4 and brine and evaporated. Loaded compound was then purified by flash chromatography with DCM(A)/EtOAc(B). Product eluted at 2% EtOAc. (*R*_{f} = 0.4 (19:1 DCM/EtOAc). Yield 573 mg (66%).

**Compound 27:** A solution of Compound 26 (250 mg, 0.15 mmol) dissolved in dry DCM (1 mL) was added to an excess of BocAmine1 (223 mg, 0.91 mmol) dissolved in dry DCM (1 mL). A solution of DMAP (37 mg, 0.62 mmol) and DIPEA (0.11 ml, 1.26 mmol) in dry DCM (1 mL) was added and the reaction mixture was stirred 16 h at 23°C under an argon atmosphere. Next day the solvent was then removed in vacuo, purified via flash chromatography on silica column (24 g) with gradient elution from 100% CH₂Cl₂ (mobile phase a) to 75:22:3 CH₂Cl₂/MeOH/NH4OHaq (by volume, mobile phase b) over 40 minutes. The desired product eluted at 53% mobile phase b. (silica gel TLC *R*_{f} = 0.6 in 1: 1 mobile phase a/mobile phase b). Yield 260 mg (75%).
**Compound 28 (PE Dendron-G2-A1-Ricinoleic. PE Dendron-G1-A1-Ricinoleic):** 100 mg of Compound 27 (0.044 mmol, MW 2290.46) was treated with 20 eq of AcCl (0.06 ml, 0.87 mmol) after dissolving the compound in 3 ml MeOH, the reaction was stirred at 0°C to 23°C for 5 h, evaporated to dryness and the crude product was dissolved in 2 ml DMF. 0.12 ml EtsN (0.87 mmol) was added followed by ricinoleic-NHS (138 mg, 0.26 mmol) dissolved in 1 ml DMF. Next day the reaction mixture was concentrated and purified via flash chromatography on silica column with gradient elution from 100% CH₂Cl₂ (mobile phase a) to 75:22:3 CH₂Cl₂/MeOH/NH₄OHaq (by volume, mobile phase b) over 40 minutes. The desired product eluted at 60% mobile phase b. (*R*_{f} = 0.7 in 1:1 mobile phase a/mobile phase b) to yield the desired product as yellow oil (98 mg, 45%). MS (ESI) calcd for C185H343N21O32 [M+3H]3+ m/z 1125.6 found 1125.2; [M+4H]4+ m/z 843.9, found 844.1.

### Example 7. Nanoparticle formulation containing Nucleic acid carrier of Formula Ia

Nanoparticles containing the PE-G2-Hexadecyl-propylpyrrolidine: DSPC: cholesterol: DMG-PEG2k at molar ratios of 1:0.5:2.4:0.04 were formulated using NanoAssemblr Benchtop (Precision NanoSystems Inc, Vancouver, BC, Canada). RNA was diluted with DNase/RNase-Free, endotoxin free distilled water and sterile citrate buffer to a final desired pH. Total flow rate was maintained at 12 mL per min at a 3:1 ratio of aqueous to organic phase for formulating on the Benchtop. Using glassware depyrogenated by heating at 250°C for 24 hour, nanoparticles were dialyzed against sterile, endotoxin-free PBS using 20,000 molecular weight cutoff dialysis. Dialyzed nanoparticles were sterile filtered using 0.2 micron poly(ether sulfone) filters and characterized with a Zetasizer NanoZS machine (Malvern). Encapsulation efficiency was measured to be 90% for the nanoparticle composition containing PE-G2-Hexadecyl-propylpyrrolidine and SEAP Replicon RNA (formulated at pH 5) using Ribogreen^{®} assay (Geall et al. 10.1073/pnas.1209367109 which is incorporated herein by reference as if fully set forth).

### Hydronamic size measurement

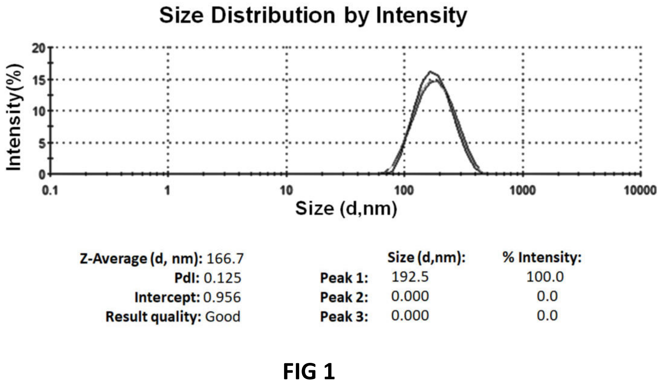

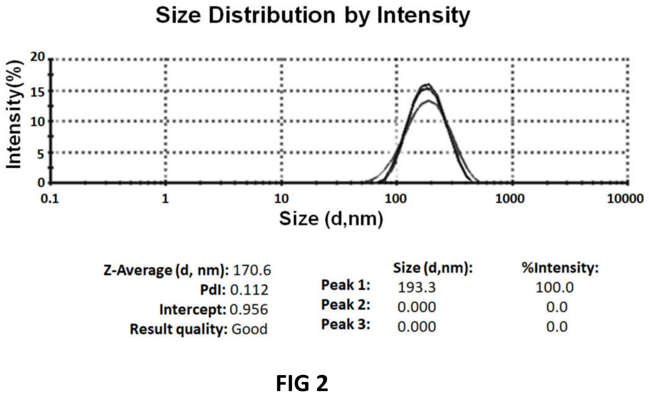

Referring to FIG. 1 and FIG. 2, the "Z average" of the nanoparticle composition containing PE-G2-Hexadecyl-propylpyrrolidine and SEAP Replicon RNA as function of size was determined by dynamic light scattering (DLS). The "Z average" is the intensity weighted mean hydrodynamic size of the ensemble collection of particles measured by dynamic light scattering (DLS). Referring to FIG. 1 the strongest intensity was observed for the nanoparticles of 192.5 d.nm in size (formulation at pH 5.0). The size distributions were characterized by a single peak with a low polydispersity index, indicating a relatively monodisperse size.

### Gel retardation assays

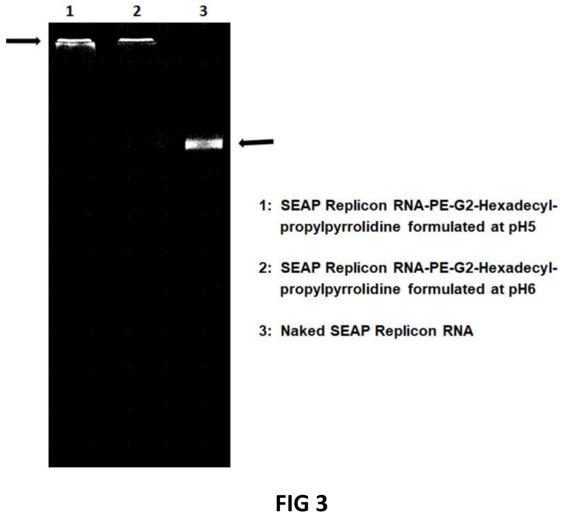

Agarose gel electrophoresis was performed to evaluate the binding of dendron carriers with RNA according to the known method (Geall et al. 10.1073/pnas.1209367109, which is incorporated herein by reference as if fully set forth). FIG. 3 is a photograph of the agarose gel showing the binding of the PE-G2-Hexadecyl-propylpyrrolidine with Replicon RNA. The gels were stained with ethidium bromide (EB) and gel images were taken on a Syngene G Box imaging system (Syngene, USA). Referring to FIG. 3, lane 1 contained the product of formulation of PE-G2-Hexadecyl-propylpyrrolidine and SEAP Replicon RNA (formulation pH 5.0), lane 2 contained the product of formulation of PE-G2-Hexadecyl-propylpyrrolidine and SEAP Replicon RNA (formulation pH 6.0) and lane 3 contained the unformulated SEAP mRNA. Before loading, the samples were incubated with formaldehyde loading dye, denatured for 10 min at 65°C and cooled to 23°C. The gel was run at 90 V and gel images were taken on a Syngene G Box imaging system (Syngene, USA). For RNA detection, the gel was stained with ethidium bromide. Referring to FIG. 3, the lower band corresponds to the small size free RNA (lane 3) and the top bands represent the large size nanoparticles formed by binding of the RNA to the nucleic acid carriers.

### In vivo SEAP Production Results.

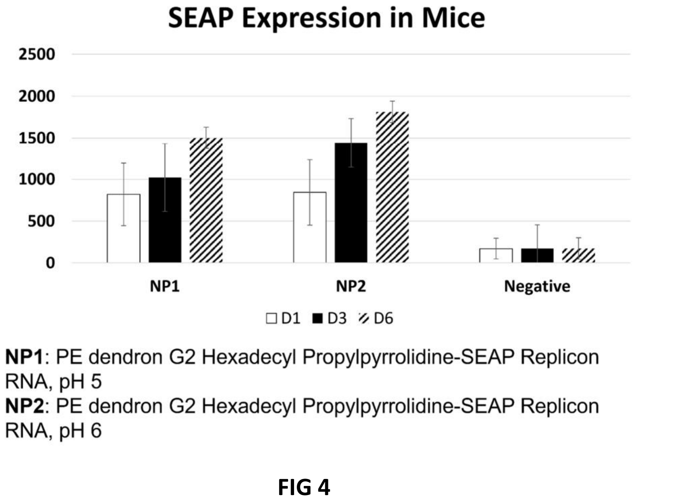

To test formulations of nucleic acid carrier of formula Ia, the secreted embryonic alkaline phosphatase SEAP reporter system was used. For *in vivo* tests, mice were injected with nanoparticles at a dose of 5 ug of SEAP Replicon RNA, and 1 day, 4 days and 6 days after administration, serum was collected from the mice. The amount of quantified using the Invitrogen NovaBright^{™} Phospha-Light^{™} EXP Assay kits for SEAP detection according to the manufacturer's protocol. The amount of SEAP in the mouse serum samples are reported in Arbitrary Units (A.U.) as measured in a BioTek Synergy HTX microplate reader. Error bars are ± S.E.M. Referring to FIG. 4, it was observed that the SEAP amount was higher with the pH 6.0 formulation, as compared to pH 5.0 because the RNA was released sooner from the pH 6.0 formulation due to weaker binding.

### Example 8. Nanoparticle Formulation containing Nucleic acid carrier of Formula Id

Nanoparticles were formulated by direct mixture of 30 µl of an ethanol phase containing Nucleic acid carrier of Formula Id (*e.g.,* PE-G2-A1-Ricinoleic.PE-G2-Linoleic) in combination with 1,2-dimyristoyl-sn-glycero-3-phosphoethanolamine-N-[methoxy(polyethyleneglycol)-2000] (PEG-lipid, Avanti Polar Lipids) with 90 µl of SEAP Replicon RNA diluted with ultraPure, DNase/RNase-free, endotoxin-free distilled water (Invitrogen) and sterile 100 mM (pH 5.0) QB Citrate Buffer (Teknova) to a final citrate concentration of 10 mM. The resulting nanoparticles contained an 16:2.3:6 mass ratio of nucleic acid carrier to PEG-lipid to Replicon RNA. Formulations were diluted 1000-fold for analysis of particle size distribution, Z-average using a Zetasizer Nano ZS (Malvern Panalytical).

### Hydronamic size measurement

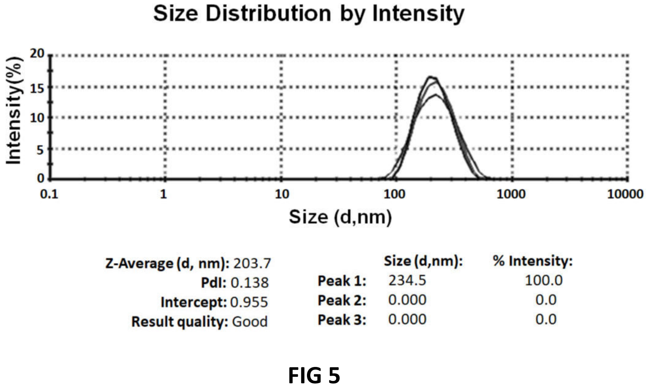

FIG. 5 illustrates distribution of the nanoparticle compositions measured as the intensity based on size (d.nm; diameter in nm) of the nanoparticles. Referring to FIG. 5, the "Z average" is the intensity weighted mean hydrodynamic size of the ensemble collection of particles measured by dynamic light scattering (DLS). Referring to FIG. 5, the strongest intensity was observed for the nanoparticles of 234.5 d.nm in size.

### Gel retardation assays

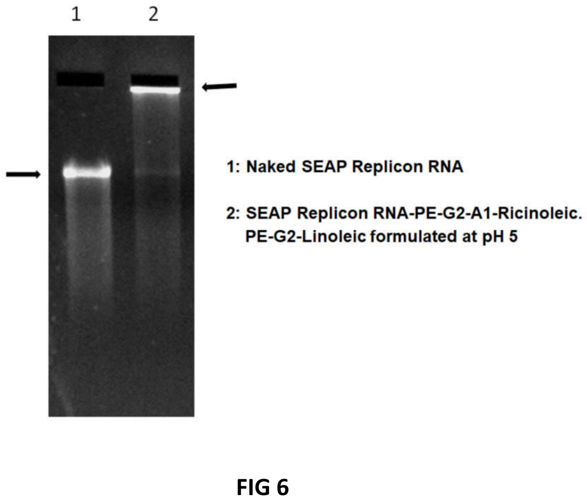

Agarose gel electrophoresis was performed to evaluate the binding of PE-G2-A1-Ricinoleic.PE-G2-Linoleic with RNA according to the known method (Geall et al. 10.1073/pnas.1209367109, which is incorporated herein by reference as if fully set forth). FIG. 6 is a photograph of the agarose gel showing the binding of the PE-G2-A1-Ricinoleic.PE-G2-Linoleic with SEAP Replicon RNA. The gels were stained with ethidium bromide (EB) and gel images were taken on a Syngene G Box imaging system (Syngene, USA). Referring to FIG. 6, lane 1 contained the unformulated SEAP RNA Replicon, lane 2 contained the product of formulation of PE-G2-A1-Ricinoleic.PE-G2-Linoleic and SEAP Replicon RNA. Before loading, the samples were incubated with formaldehyde loading dye, denatured for 10 min at 65°C and cooled to 23°C. The gel was run at 90 V and gel images were taken on a Syngene G Box imaging system (Syngene, USA). For RNA detection, the gel was stained with ethidium bromide. Referring to FIG. 6, the lower band corresponds to the small size free RNA (lane 1) and the top band represent the large size nanoparticles formed by binding of the RNA to the nucleic acid carrier (lane 2).

### In vitro SEAP Production Results.

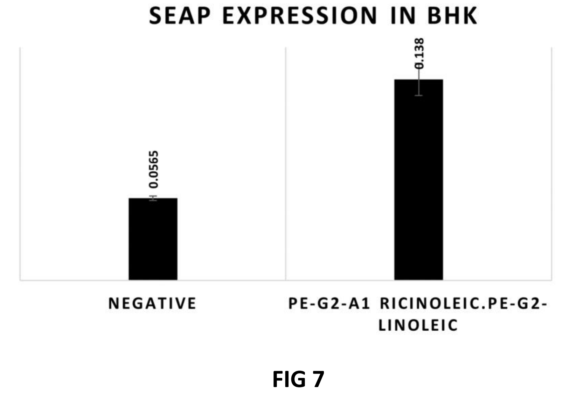

To test the ability of above nanoparticle composition to express SEAP *in vitro,* BHK cells were treated with nanoparticles. Each well of a 12 well dish of BHK was treated with 10 µL (approximately 1 µg) of formulation product diluted into a final volume of 500 µL with a 1:1 Optimem:PBS mix. The nontreated well had 500 µL of 50/50 PBS/OptiMEM. Twenty four hours posttreatment or transfection, conditioned media from each culture was collected. Media was analyzed using the QuantiBlue assay (InvivoGen). 150 µL QuantiBlue reagent was combined with 50 µL of media. Readings were taken after 10 minutes by measuring absorbance at 650 nm as directed by the manufacturer's protocol. For the no treatment sample or negative control, 50 µL of media from a well not treated with nanoparticles was added to 150 µL QuantiBlue Reagent. FIG. 7 illustrates *in vitro* SEAP expression of nanoparticle formulation containing PE-G2-A1-Ricinoleic.PE-G2-Linoleic as nucleic acid carrier.

### Example 9. Nanoparticle formulation containing Nucleic acid carrier of Formula If

Nanoparticles containing the PE-G1-A1-Ricinoleic. PE-G1-A1-Ricinoleic or PE-G2-A1-Ricinoleic. PE-G1-A1-Ricinoleic: DOPE: cholesterol: DMG-PEG2k at molar ratios of 1:0.6:2.88:0.1 were formulated using NanoAssemblr Benchtop (Precision NanoSystems Inc, Vancouver, BC, Canada). RNA was diluted with DNase/RNase-Free, endotoxin free distilled water and sterile citrate buffer to a final desired pH. Total flow rate was maintained at 10 mL per min at a 3:1 ratio of aqueous to organic phase for formulating on the Benchtop. Using glassware depyrogenated by heating at 250°C for 24 hour, nanoparticles were dialyzed against sterile, endotoxin-free PBS using 20,000 molecular weight cutoff dialysis. Dialyzed nanoparticles were sterile filtered using 0.2 micron poly(ether sulfone) filters and characterized with a Zetasizer NanoZS machine (Malvern). Encapsulation efficiency was measured to be 93% and 90% for the nanoparticle composition containing PEG1-A1-Ricinoleic. PE-G1-A1-Ricinoleic or PE-G2-A1-Ricinoleic. PE-G1-A1-Ricinoleic and SEAP Replicon RNA (formulated at pH 6) using Ribogreen^{®} assay (Geall et al. 10.1073/pnas.1209367109 which is incorporated herein by reference as if fully set forth).

### Hydronamic size measurement

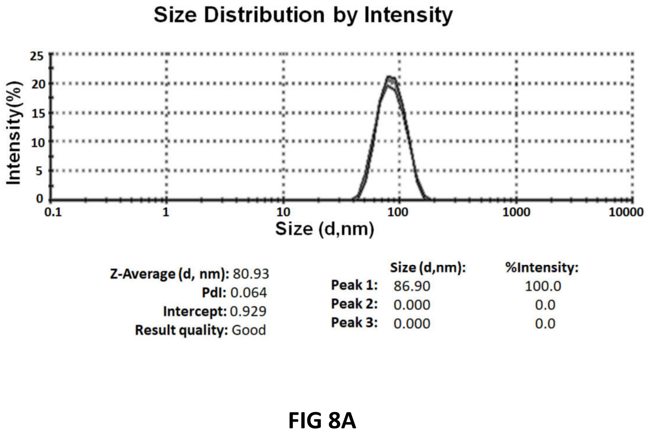

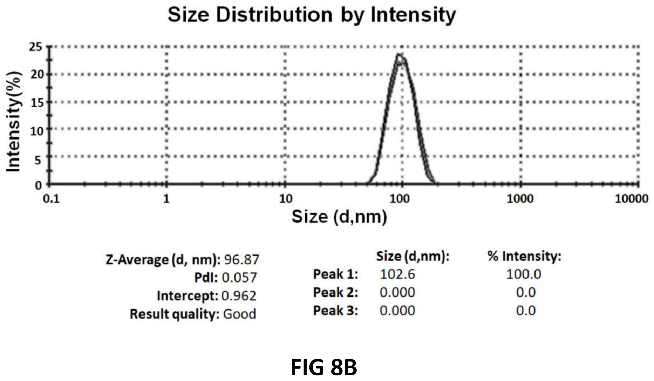

FIG. 8A and FIG. 8B illustrates distribution of the nanoparticle composition measured as the intensity based on size (d.nm; diameter in nm) of the nanoparticles. Referring to FIG. 8A and FIG. 8B the strongest intensity was observed for the nanoparticles of 86.90 d.nm in size and 102.6 d.nm in size respectively. The size distributions were characterized by a single peak with a low polydispersity index, indicating a relatively monodisperse size.

### In vitro SEAP Production Results.

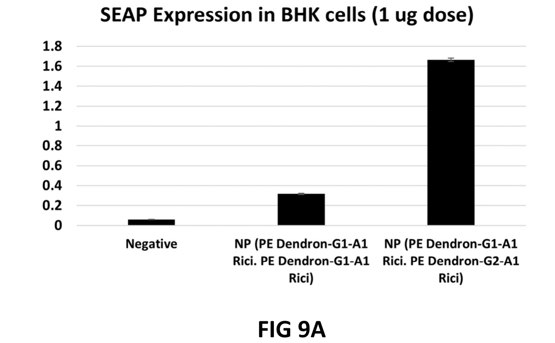

To test the ability of above nanoparticle compositions to express SEAP *in vitro,* BHK cells were treated with nanoparticles. Each well of a 12 well dish of BHK was treated with 10 µL (approximately 1 µg) of formulation product diluted into a final volume of 500 µL with a 1:1 Optimem:PBS mix. The nontreated well had 500 µL of 50/50 PBS/OptiMEM. Twenty-four hours posttreatment or transfection, conditioned media from each culture was collected. Media was analyzed using the QuantiBlue assay (InvivoGen). 150 µL QuantiBlue reagent was combined with 50 µL of media. Readings were taken after 10 minutes by measuring absorbance at 650 nm as directed by the manufacturer's protocol. For the no treatment sample or negative control, 50 µL of media from a well not treated with nanoparticles was added to 150 µL QuantiBlue Reagent. FIG. 9A illustrates *in vitro* SEAP expression of nanoparticle formulation containing PE-G1-A1-Ricinoleic. PE-G1-A1-Ricinoleic or PE-G2-A1-Ricinoleic. PE-G1-A1-Ricinoleic as nucleic acid carriers.

### In vivo SEAP Production Results.

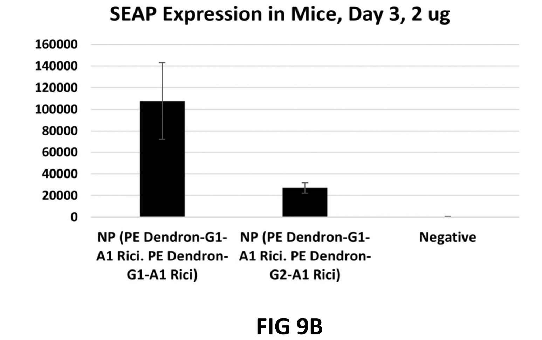

To test formulations with PE-G1-A1-Ricinoleic. PE-G1-A1-Ricinoleic or PE-G2-A1-Ricinoleic. PE-G1-A1-Ricinoleic *in vivo*, mice were injected with nanoparticles at a dose of 2 ug of SEAP Replicon RNA, and 3 days after administration, serum was collected from the mice. The amount of quantified using the Invitrogen NovaBright^{™} Phospha-Light^{™} EXP Assay kits for SEAP detection according to the manufacturer's protocol. The amount of SEAP in the mouse serum samples are reported in Arbitrary Units (A.U.) as measured in a BioTek Synergy HTX microplate reader. Error bars are ± S.E.M. Referring to FIG. 9B, it was observed that the SEAP amount was higher with PE-G1-A1-Ricinoleic. PE-G1-A1-Ricinoleic, as compared to PE-G2-A1-Ricinoleic. PE-G1-A1-Ricinoleic.

### COVID-19 Wuhan Spike RBD Direct Serum ELISA

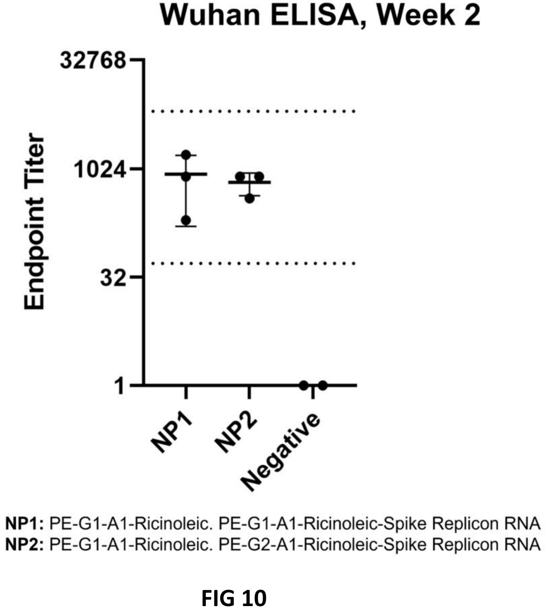

Mice (BALB/c) were vaccinated by IM injection bilaterally in the leg muscle with 2 µg of Spike Replicon RNA formulated with PE-G1-A1-Ricinoleic. PE-G1-A1-Ricinoleic and PE-G2-A1-Ricinoleic. PE-G1-A1-Ricinoleic (in a total volume of 100 µL PBS). Mice were bled 14 days post injection, and serum was isolated from whole blood by centrifuging coagulated samples at 10000 RCF for 1.5 minutes. This serum was assayed for anti-Spike RBD antibody titer by direct ELISA. Greiner Bio High Binding Microlon ELISA plates were coated overnight with PBS containing recombinant Wuhan Spike RBD protein at 4°C. Wells were blocked with PBS + 0.5% BSA for 1 hour at room temperature. Serum samples was added to the wells starting at a 1:50 dilution and with serial 1:2 dilutions up to 1:6400 in PBS+ 0.5% BSA. Samples were incubated at RT for 1 hour, washed 3 times with PBST, and then goat antimouse IgG HRP was added at a 1:20,000 dilution in PBS + 0.5% BSA and incubated for 1 hour. Plates were again washed 5 times with PBST and developed using the chromogenic HRP substrate 3,3',5,5'-Tetramethylbenzidine (TMB). The reaction was stopped by addition of 0.5M H2SO4 and absorbance was measured at 450nm and 570nm. Endpoint titer was designated as the highest dilution at which the value of Absorbance at 450 - Absorbance at 570 was ≥ 0.08. At week 2, the endpoint dilution titers for groups immunized with 2 µg of nanoparticle vaccine formulated using PE-G1-A1-Ricinoleic. PE-G1-A1-Ricinoleic and PE-G2-A1-Ricinoleic. PE-G1-A1-Ricinoleic (FIG. 10) were between200 and 1600., This is in contrast to the seronegative group not immunized wherein the titer remained at the baseline.

### References:

The references cited throughout this application, are incorporated for all purposes apparent herein and in the references themselves as if each reference was fully set forth. For the sake of presentation, specific ones of these references are cited at particular locations herein. A citation of a reference at a particular location indicates a manner(s) in which the teachings of the reference are incorporated. However, a citation of a reference at a particular location does not limit the manner in which all of the teachings of the cited reference are incorporated for all purposes.
**(1)** (a) Jones, C. H., Chen, C.-K., Ravikrishnan, A., Rane, S., and Pfeifer, B. A. (2013) Overcoming nonviral gene delivery barriers: perspective and future. Mol. Pharmaceutics 10, 4082-4098. (b) Gomes, C. P., Lopes, C. D. F., Moreno, P. M. D., Varela-Moreira, A., Alonso, M. J., and Pego, A. P. (2014) Translating chitosan to clinical delivery of nucleic acid-based drugs. MRS Bull. 39, 60-70. (c) Mendes, L.P., Pan, J., Torchilin, V.P. (2017) Dendrimers as Nanocarriers for Nucleic Acid and Drug Delivery in Cancer Therapy. Molecules 22(9), 1401. (d) Nishikawa, M., and Huang, L. (2001) Nonviral vectors in the new millennium: delivery barriers in gene transfer. Hum. Gene Ther. 12, 861-87. (e) Clare, E. T., Anja, E., and Mark, A. K. (2003) Progress and problems with the use of viral vectors for gene therapy. Nat. Rev. Genet. 4, 346-358. (f) Mintzer, M. A., and Simanek, E. E. (2009) Nonviral Vectors for Gene Delivery. Chem. Rev. 109, 259-302.
**(2)** (a) Hassett KJ, Benenato KE, Jacquinet E, Lee A, Woods A, Yuzhakov O, Himansu S, Deterling J, Geilich BM, Ketova T, Mihai C, Lynn A, McFadyen I, Moore MJ, Senn JJ, Stanton MG, Almarsson Ö, Ciaramella G, Brito LA. Optimization of Lipid Nanoparticles for Intramuscular Administration of mRNA Vaccines. Mol Ther Nucleic Acids. 2019 Apr 15;15:1-11. doi: 10.1016/j.omtn.2019.01.013. Epub 2019 Feb 7. PMID: 30785039; PMCID: PMC6383180. (b)Reichmuth AM, Oberli MA, Jaklenec A, Langer R, Blankschtein D. mRNA vaccine delivery using lipid nanoparticles [published correction appears in Ther Deliv. 2016 Jun; 7(6):411]. Ther Deliv. 2016;7(5):319-334. doi:10.4155/tde-2016-0006.
**(3)** (a) Welsh, D. J., Jones, S. P., and Smith, D. K. (2009) "On-off' multivalent recognition: degradable dendrons for temporary highaffinity DNA binding. Angew. Chem., Int. Ed. 48, 4047-4051.
**(4)** (a) Welsh, D. J., Jones, S. P., and Smith, D. K. (2009) "On-off' multivalent recognition: degradable dendrons for temporary highaffinity DNA binding. Angew. Chem., Int. Ed. 48, 4047-4051.
   (b) Barnard, A., Posocco, P., Pricl, S., Calderon, M., Haag, R., Hwang, M. E., Shum, V. W., Pack, D. W., and Smith, D. K. (2011) Degradable self-assembling dendrons for gene delivery: experimental and theoretical insights into the barriers to cellular uptake. J. Am. Chem. Soc. 133, 20288-20300.
**(5)** (a) M.A. Carnahan, M.W. Grinstaff, J. Am. Chem. Soc. 123 (2001) 2905. (b) M.A. Carnahan, M.W. Grinstaff, Macromolecules 34 (2001) 7648. (c) M.A. Carnahan, M.W. Grinstaff, Macromolecules 39 (2006) 609.
**(6)** LUNDBERG, A., KULKARNI, S., KLEIN, L., PADMANABHAN, H.K. ARATYN, Y.S. COMPOSITIONS AND METHODS FOR GENE EDITING, WO/2018/154387.
**(7)** Geall AJ, Verma A, Otten GR, Shaw CA, Hekele A, Banerjee K, Cu Y, Beard CW, Brito LA, Krucker T, O'Hagan DT, Singh M, Mason PW, Valiante NM, Dormitzer PR, Barnett SW, Rappuoli R, Ulmer JB, Mandl CW. Nonviral delivery of self-amplifying RNA vaccines. Proc Natl Acad Sci U S A. 2012 Sep 4;109(36):14604-9. doi: 10.1073/pnas.1209367109. Epub 2012 Aug 20. PMID: 22908294; PMCID: PMC3437863.
**(9)** Poulami Talukder, Jasdave S. Chahal, Justine S. McPartlan, Omar Khan, Karl Ruping. Nanoparticle Compositions for Efficient Nucleic Acid Delivery and Methods of Making and Using the Same. WO 2020/132196 (PCT/US19/67402).
**(10)** Mui, Barbara L et al. "Influence of Polyethylene Glycol Lipid Desorption Rates on Pharmacokinetics and Pharmacodynamics of siRNA Lipid Nanoparticles." Molecular therapy. Nucleic acids vol. 2,12 e139. 17 Dec. 2013, doi:10.1038/mtna.2013.66
The invention will now be described by the following non-limiting sequentially numbered statements statements :
1. A nucleic acid carrier comprising a dendron or dendrimer of Formula Ia, Ib, Ic, Id, Ie, or If: 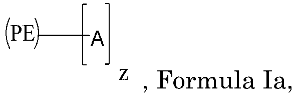 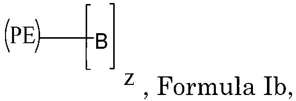 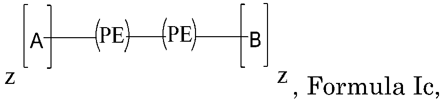 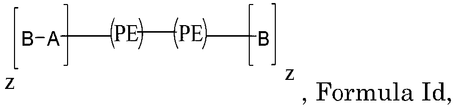 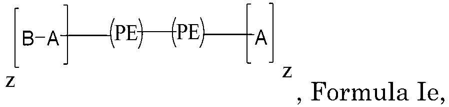 or 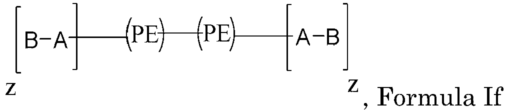 wherein PE is a polyester dendron, which includes a core and a plurality of monomeric polyester units that form one or more generations, A is an amine, B is a hydrophobic unit, and z is the number of surface groups.
2. The nucleic acid carrier of statement 1, wherein PE has the Formula II:

   [(core)-Gn-O], II,

   wherein G is a layer or generation of dendron and n is a generation number and is in a range from 1 to 10.
3. The nucleic acid carrier of statement 1, wherein the plurality of monomeric polyester units are 2,2-bis(hydroxymethyl) propionic acid or 2,2-bis(hydroxymethyl)butyric acid.
4. The nucleic acid carrier of statement 1, wherein z has Formula III:

   z = bⁿ, III,

   wherein b is branch point multiplicity, or number of branches at each branching point, and n is a generation number and is in a range from 1 to 10.
5. The nucleic acid carrier of statement 1, wherein A is 1-(2-Aminoethyl)piperazine, 1-Piperidineethanamine, 1-Dimethylamino-2-propylamine, 1-(3-Aminopropyl)pyrrolidine, 1-(2-Aminoethyl)pyrrolidine, 1-(2-Aminoethyl)piperidine, 2-(1-Piperazinyl)ethylamine, 2-(4-Methyl-piperazin-1-yl)-ethylamine, 3-(Dimethylamino)-1-propylamine, 3-(Diethylamino)propylamine, (4-Aminobutyl)dimethylamine, 4-(1-Pyrrolidinyl)-1-butylamine, 4-Morpholineethanamine, 4-Morpholinepropanamine, 4-Methyl-1-piperazineethanamine, 4-(Diethylamino)butylamine, N,N-Dimethylethylenediamine, N,N-Dimethyldipropylenetriamine, N,N'-Dimethyl-N,N'-bis(3-methylaminopropyl)trimethylenediamine, N1-(3-aminopropyl)propane-1,3-diamine, N1-(3-aminopropyl)-N1-methylpropane-1,3-diamine, 3-((3-aminopropyl)(methyl)amino)propan-1-ol, 1-Dimethylamino-2-propanol, 1-[Bis[3-(dimethylamino)propyl]amino]-2-propanol, 2-Dimethylaminoethanol, 2-(Diethylamino)ethanol, 2-[2-(Dimethylamino)ethoxy]ethanol, 2-(2-Aminoethoxy)ethanol, 2-{[2-(Dimethylamino)ethyl]methylamino}ethanol, 3-Dimethylamino-1-propanol, 3-Diethylamino-1-propanol, 4-(Dimethylamino)-1-butanol, 4-Diethylamino-2-butyn-1-ol, N-Methyldiethanolamine, 4-(diethylamino)butan-1-ol, 3-(azetidin-1-yl)propan-1-ol, 3-(pyrrolidin-1-yl)propan-1-ol, 3-(piperidin-1-yl)propan-1-ol, 4-(azetidin-1-yl)butan-1-ol, 4-(pyrrolidin-1-yl)butan-1-ol, 4-(piperidin-1-yl)butan-1-ol, 3-morpholinopropan-1-ol, 4-morpholinobutan-1-ol, 3-(4-methylpiperazin-1-yl)propan-1-ol, or 4-(4-methylpiperazin-1-yl)butan-1-ol.
6. The nucleic acid carrier of any one of the previous statements, wherein B is derived from a fatty acid (C4-C28) or derivative thereof.
7. The nucleic acid carrier of statement 6, wherein the fatty acid is selected from the group consisting of: caprylic acid, capric acid, lauric acid, myristic acid, palmitic acid, stearic acid, oleic acid, linoleic acid, linolenic acid, arachidonic acid, and eicosapentanoic acid.
8. The nucleic acid carrier of statement 6, wherein the fatty acid derivative is selected from the group consisting of: 12-hydroxy-9-cis-octadecenoic acid, 12-methyltetradecanoic acid, 12-methyltridecanoic acid, 14-methylhexadecanoic acid, 14-methylhexadecanoic acid, 18-methylnonadecanoic acid, 19-methylarachidic acid, isopalmitic acid, isostearic acid, phytanic acid, (±)-2-hydroxyoctanoic acid, (±)-3-hydroxydecanoic acid, (±)-3-hydroxyoctanoic acid, 10-hydroxydecanoic acid, 12-hydroxyoctadecanoic acid, 15-hydroxypentadecanoic acid, 16-hydroxyhexadecanoic acid, 2-hydroxyhexadecanoic acid, 2-hydroxytetradecanoic acid, 2-hydroxydodecanoic acid, DL-α-hydroxystearic acid, DL-β-hydroxylauric acid, DL-β-hydroxymyristic acid, and DL-6-hydroxypalmitic acid.
9. The nucleic acid carrier of statement 6, wherein the fatty acid or derivative comprises one or more stable isotopes.
10. The nucleic acid carrier of statement 9, wherein the stable isotope is a stable isotope of carbon or hydrogen.
11. The nucleic acid carrier of statement 10, wherein the stable isotope of carbon is ¹³C.
12. The nucleic acid carrier of statement 10, wherein the stable isotope of hydrogen is ²H.
13. The nucleic acid carrier of statement 10, wherein the fatty acid that comprises the stable isotope is selected from the group consisting of: octanoic acid-1-13C, octanoic acid-8-13C, octanoic acid-8,8,8-d3, octanoic-2H15 acid, decanoic acid-1-13C, decanoic acid-10-13C, decanoic-10,10,10-d3 acid, decanoic-d19 acid, undecanoic acid-1-13C, lauric acid-12,12,12-2H3, lauric-2H23 acid, lauric acid-1-13C, lauric acid-1,12-13C2, tridecanoic-2,2-2H2 acid, myristic acid-14-13C, myristic acid-1-13C, myristic acid-14,14,14-2H3, myristic-2H27 acid, palmitic acid-1-13C, palmitic acid-16-13C, palmitic acid-16-13C,16,16,16-2H3, palmitic acid-2H31, stearic acid-1-13C, stearic acid-18-13C, stearic acid-18,18,18-2H3, stearic-2H35 acid, oleic acid-1-13C, oleic acid-2H34, linolenic acid-1-13C, linoleic acid-2H32, arachidonic-5,6,8,9,11,12,14,15-2H8 acid, and eicosanoic-2H39 acid.
14. A nanoparticle composition comprising the nucleic acid carrier of any one of statements 1-13, and a therapeutic or immunogenic nucleic acid agent fully or partially encapsulated therein.
15. The nanoparticle composition of statement 14, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.
16. The nanoparticle composition of statement 14, wherein the therapeutic or immunogenic nucleic acid agent encodes one or more antigens selected from the group consisting of infectious disease, pathogen, cancer, autoimmunity disease and allergenic disease.
17. The nanoparticle composition of statement 14, wherein the therapeutic or immunogenic nucleic acid agent comprises an RNA or DNA capable of silencing, inhibiting or modifying the activity of a gene.
18. The nanoparticle composition of statement 14 further comprising a PEG-lipid.
19. The nanoparticle composition of statement 18, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.
20. The nanoparticle composition of statement 18, wherein the therapeutic or immunogenic nucleic acid agent encodes one or more antigens selected from the group consisting of infectious disease, pathogen, cancer, autoimmunity disease and allergenic disease.
21. The nanoparticle composition of statement 18, wherein the therapeutic or immunogenic nucleic acid agent comprises an RNA or DNA capable of silencing, inhibiting or modifying the activity of a gene.
22. The nanoparticle composition of statement 18, wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000.
23. The nanoparticle composition of statement 18, wherein the nanoparticle composition comprises the PEG-lipid in a range from 1 mol% to 10 mol% of the PEG-lipid per nanoparticle composition.
24. The nanoparticle composition of statements 18 further comprising a phospholipid and cholesterol or derivative thereof.
25. The nanoparticle composition of statement 24, wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000.
26. The nanoparticle composition of statement 24, wherein the nanoparticle composition comprises the PEG-lipid in a range from 1 mol% to 10 mol% of the PEG-lipid per nanoparticle composition.
27. The nanoparticle composition of statement 24, wherein the phospholipid is 1,2-dioleoyl-sn-glycero-3-phosphoethanolamine (DOPE) or distearoylphosphatidylcholine (DSPC).
28. The nanoparticle composition of statement 24, wherein the nanoparticle composition comprises the phospholipid in a range from 10 mol% to 15 mol% of the phospholipid per nanoparticle composition.
29. The nanoparticle composition of statement 24, wherein the nanoparticle composition comprises the cholesterol or derivative thereof in a range from 50 mol% to 75 mol% of the cholesterol or derivative thereof per nanoparticle composition.
30. A method for treating or preventing a disease or condition in a subject comprising: administering a therapeutically effective amount of the nanoparticle composition of any one of statement 14to a subject.
31. The method of statement 30, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.
32. The method of statement 30, wherein the therapeutic or immunogenic nucleic acid agent encodes one or more antigens selected from the group consisting of infectious disease, pathogen, cancer, autoimmunity disease and allergenic disease.
33. The method of statement 30, wherein the therapeutic or immunogenic nucleic acid agent comprises an RNA or DNA capable of silencing, inhibiting or modifying the activity of a gene.
34. The method of statement 30, wherein that nanoparticle composition further comprises a PEG-lipid.
35. The nanoparticle composition of statement 34, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.
36. The nanoparticle composition of statement 34, wherein the therapeutic or immunogenic nucleic acid agent encodes one or more antigens selected from the group consisting of infectious disease, pathogen, cancer, autoimmunity disease and allergenic disease.
37. The nanoparticle composition of statement 34, wherein the therapeutic or immunogenic nucleic acid agent comprises an RNA or DNA capable of silencing, inhibiting or modifying the activity of a gene.
38. The nanoparticle composition of statement 34, wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000.
39. The nanoparticle composition of statement 34, wherein the nanoparticle composition comprises the PEG-lipid in a range from 1 mol% to 10 mol% of the PEG-lipid per nanoparticle composition.
40. The nanoparticle composition of statements 39, wherein the nanoparticle composition further comprises a phospholipid and cholesterol or derivative thereof.
41. The nanoparticle composition of statement 40, wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000.
42. The nanoparticle composition of statement 40, wherein the nanoparticle composition comprises the PEG-lipid in a range from 1 mol% to 10 mol% of the PEG-lipid per nanoparticle composition.
43. The nanoparticle composition of statement 40, wherein the phospholipid is 1,2-dioleoyl-sn-glycero-3-phosphoethanolamine (DOPE) or distearoylphosphatidylcholine (DSPC).
44. The nanoparticle composition of statement 40, wherein the nanoparticle composition comprises the phospholipid in a range from 10 mol% to 15 mol% of the phospholipid per nanoparticle composition.
45. The nanoparticle composition of statement 40, wherein the nanoparticle composition comprises the cholesterol or derivative thereof in a range from 50 mol% to 75 mol% of the cholesterol or derivative thereof per nanoparticle composition.
46. The method of statement 30, wherein the therapeutically effective amount of the nanoparticle composition comprises the therapeutic or immunogenic nucleic acid agent in a range from 0.01 mg nucleic acid to 10 mg nucleic acid per kg body weight of the subject.
47. The method of statement 46, wherein the subject is a mammal.
48. The method of statement 47, wherein the mammal is selected from the group consisting of: a rodent, a canine, a primate, an equine, a high value agricultural animal, and a human.

## Claims

1. A nucleic acid carrier comprising a dendron or dendrimer of Formula Ic, Id, Ie, or If: 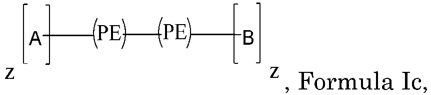 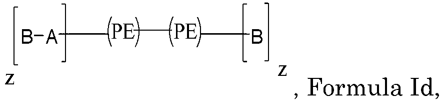 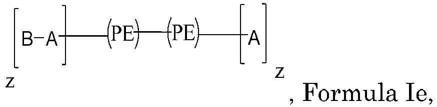 or 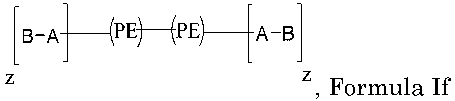 wherein PE is a polyester dendron, which includes a core and a plurality of monomeric polyester units that form one or more generations, A is an amine, B is a hydrophobic unit, and z is the number of surface groups.

2. The nucleic acid carrier of claim 1, wherein PE has the Formula II:
[(core)-Gn-O], II,
wherein G is a layer or generation of dendron and n is a generation number and is in a range from 1 to 10.

3. The nucleic acid carrier of claim 1, wherein the plurality of monomeric polyester units are 2,2-bis(hydroxymethyl) propionic acid or 2,2-bis(hydroxymethyl)butyric acid.

4. The nucleic acid carrier of claim 1, wherein z has Formula III:
z = bⁿ, III,
wherein b is branch point multiplicity, or number of branches at each branching point, and n is a generation number and is in a range from 1 to 10.

5. The nucleic acid carrier of claim 1, wherein A is 1-(2-Aminoethyl)piperazine, 1-Piperidineethanamine, 1-Dimethylamino-2-propylamine, 1-(3-Aminopropyl)pyrrolidine, 1-(2-Aminoethyl)pyrrolidine, 1-(2-Aminoethyl)piperidine, 2-(1-Piperazinyl)ethylamine, 2-(4-Methyl-piperazin-1-yl)-ethylamine, 3-(Dimethylamino)-1-propylamine, 3-(Diethylamino)propylamine, (4-Aminobutyl)dimethylamine, 4-(1-Pyrrolidinyl)-1-butylamine, 4-Morpholineethanamine, 4-Morpholinepropanamine, 4-Methyl-1-piperazineethanamine, 4-(Diethylamino)butylamine, N,N-Dimethylethylenediamine, N,N-Dimethyldipropylenetriamine, N,N'-Dimethyl-N,N'-bis(3-methylaminopropyl)trimethylenediamine, N1-(3-aminopropyl)propane-1,3-diamine, N1-(3-aminopropyl)-N1-methylpropane-1,3-diamine, 3-((3-aminopropyl)(methyl)amino)propan-1-ol, 1-Dimethylamino-2-propanol, 1-[Bis[3-(dimethylamino)propyl]amino]-2-propanol, 2-Dimethylaminoethanol, 2-(Diethylamino)ethanol, 2-[2-(Dimethylamino)ethoxy]ethanol, 2-(2-Aminoethoxy)ethanol, 2-{[2-(Dimethylamino)ethyl]methylamino}ethanol, 3-Dimethylamino-1-propanol, 3-Diethylamino-1-propanol, 4-(Dimethylamino)-1-butanol, 4-Diethylamino-2-butyn-1-ol, N-Methyldiethanolamine, 4-(diethylamino)butan-1-ol, 3-(azetidin-1-yl)propan-1-ol, 3-(pyrrolidin-1-yl)propan-1-ol, 3-(piperidin-1-yl)propan-1-ol, 4-(azetidin-1-yl)butan-1-ol, 4-(pyrrolidin-1-yl)butan-1-ol, 4-(piperidin-1-yl)butan-1-ol, 3-morpholinopropan-1-ol, 4-morpholinobutan-1-ol, 3-(4-methylpiperazin-1-yl)propan-1-ol, or 4-(4-methylpiperazin-1-yl)butan-1-ol.

6. The nucleic acid carrier of any one of the previous claims, wherein B is derived from a fatty acid (C4-C28) or derivative thereof;
preferably wherein the fatty acid is selected from the group consisting of: caprylic acid, capric acid, lauric acid, myristic acid, palmitic acid, stearic acid, oleic acid, linoleic acid, linolenic acid, arachidonic acid, and eicosapentanoic acid;
more preferably wherein the fatty acid derivative is selected from the group consisting of: 12-hydroxy-9-cis-octadecenoic acid, 12-methyltetradecanoic acid, 12-methyltridecanoic acid, 14-methylhexadecanoic acid, 14-methylhexadecanoic acid, 18-methylnonadecanoic acid, 19-methylarachidic acid, isopalmitic acid, isostearic acid, phytanic acid, (±)-2-hydroxyoctanoic acid, (±)-3-hydroxydecanoic acid, (±)-3-hydroxyoctanoic acid, 10-hydroxydecanoic acid, 12-hydroxyoctadecanoic acid, 15-hydroxypentadecanoic acid, 16-hydroxyhexadecanoic acid, 2-hydroxyhexadecanoic acid, 2-hydroxytetradecanoic acid, 2-hydroxydodecanoic acid, DL-α-hydroxystearic acid, DL-β-hydroxylauric acid, DL-β-hydroxymyristic acid, and DL-β-hydroxypalmitic acid.

7. The nucleic acid carrier of claim 6, wherein the fatty acid or derivative comprises one or more stable isotopes;
preferably wherein the stable isotope is a stable isotope of carbon or hydrogen;
more preferably wherein the stable isotope of carbon is ¹³C;
even more preferably wherein the stable isotope of hydrogen is ²H.

8. The nucleic acid carrier of claim 7, wherein the fatty acid that comprises the stable isotope is selected from the group consisting of: octanoic acid-1-13C, octanoic acid-8-13C, octanoic acid-8,8,8-d3, octanoic-2H15 acid, decanoic acid-1-13C, decanoic acid-10-13C, decanoic-10,10,10-d3 acid, decanoic-d19 acid, undecanoic acid-1-13C, lauric acid-12,12,12-2H3, lauric-2H23 acid, lauric acid-1-13C, lauric acid-1,12-13C2, tridecanoic-2,2-2H2 acid, myristic acid-14-13C, myristic acid-1-13C, myristic acid-14,14,14-2H3, myristic-2H27 acid, palmitic acid-1-13C, palmitic acid-16-13C, palmitic acid-16-13C,16,16,16-2H3, palmitic acid-2H31, stearic acid-1-13C, stearic acid-18-13C, stearic acid-18,18,18-2H3, stearic-2H35 acid, oleic acid-1-13C, oleic acid-2H34, linolenic acid-1-13C, linoleic acid-2H32, arachidonic-5,6,8,9,11,12,14,15-2H8 acid, and eicosanoic-2H39 acid.

9. A nanoparticle composition comprising the nucleic acid carrier of any one of claims 1-8, and a therapeutic or immunogenic nucleic acid agent fully or partially encapsulated therein.

10. The nanoparticle composition of claim 9, wherein the therapeutic or immunogenic nucleic acid agent is selected from the group consisting of: a polynucleotide, oligonucleotide, DNA, cDNA, RNA, repRNA, siRNA, miRNA, sgRNA, and mRNA.

11. The nanoparticle composition of claim 9, wherein the therapeutic or immunogenic nucleic acid agent encodes one or more antigens selected from the group consisting of infectious disease, pathogen, cancer, autoimmunity disease and allergenic disease.

12. The nanoparticle composition of claim 9, wherein the therapeutic or immunogenic nucleic acid agent comprises an RNA or DNA capable of silencing, inhibiting or modifying the activity of a gene.

13. The nanoparticle composition of claim 9 further comprising a PEG-lipid;
optionally wherein the PEG-lipid is 1,2-dimyristoyl-sn-glycero-3-phosphoethanolamine-N-[methoxy (poly- ethylene glycol)-2000] or 1,2-dimyristoyl-rac-glycero-3-methoxypolyethylene glycol-2000;
further optionally wherein the nanoparticle composition comprises the PEG-lipid in a range from 1 mol% to 10 mol% of the PEG-lipid per nanoparticle composition.

14. The nanoparticle composition of claim 13 further comprising a phospholipid and cholesterol or derivative thereof;
optionally wherein the phospholipid is 1,2-dioleoyl-sn-glycero-3-phosphoethanolamine (DOPE) or distearoylphosphatidylcholine (DSPC);
further optionally wherein the nanoparticle composition comprises the phospholipid in a range from 10 mol% to 15 mol% of the phospholipid per nanoparticle composition;
still further optionally wherein the nanoparticle composition comprises the cholesterol or derivative thereof in a range from 50 mol% to 75 mol% of the cholesterol or derivative thereof per nanoparticle composition.

15. A therapeutically effective amount of the nanoparticle composition according to any one of the preceding claims for use as a medicament;
preferably wherein the therapeutically effective amount of the nanoparticle composition comprises the therapeutic or immunogenic nucleic acid agent in a range from 0.01 mg nucleic acid to 10 mg nucleic acid per kg body weight of the subject;
optionally wherein the subject is a mammal;
still optionally wherein the mammal is selected from the group consisting of: a rodent, a canine, a primate, an equine, a high value agricultural animal, and a human.
